# EUROPEAN PATENT APPLICATION

(11) **EP 4 700 036 A1**
(43) Date of publication of application: **25.02.2026**
(21) Application number: 24792772.6
(22) Date of filing: 19.04.2024
(51) Int. Cl.: C07H 15/203, A61K 31/7034, A61K 39/39, A61P 31/04, A61P 31/12, A61P 35/00, A61P 37/04, A61P 43/00

(54) **GLYCOLIPID DERIVATIVE AS MINCLE LIGAND**

(30) Priority: 20.04.2023 JP 2023069661
(71) Applicant: The University of Osaka, Osaka 565-0871 (JP)
(72) Inventor: YAMASAKI, Sho, Suita-shi, Osaka 565-0871 (JP); ISHIZUKA, Shigenari, Suita-shi, Osaka 565-0871 (JP)
(74) Representative: Mewburn Ellis LLP
(86) International application number: PCT/JP2024/015647
(87) International publication number: WO 2024/219501

(57) **Abstract**

In the present application, glycolipid derivative (I) represented by the following formula (I): 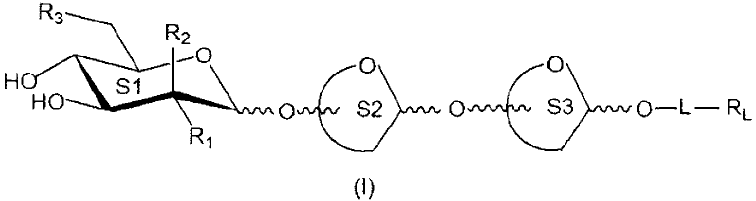 wherein each symbol is as described in the specification, or a salt thereof, which is useful as a novel ligand for Macrophage-inducible C-type lectin receptors, is disclosed as one of the embodiments of the invention.

## Description

### [Technical Field]

The present invention relates to a glycolipid derivative and the like that are ligands for Macrophage-inducible C-type lectin receptors, that play an important role in immune responses, and is useful in the pharmaceutical field.

### [Background Art]

The existence of compounds that activate immune responses, represented by Freund's adjuvant, has long been known. However, in recent years, it has become clear that ligand recognition by pattern-recognition receptors (PRRs) enhances antigen-specific antibody production and the induction of immune memory. While these compounds have a potent immunostimulatory action, many are toxic, and only a small number of the compounds are clinically applied.

Among PRRs, the macrophage-inducible C-type lectin receptor (hereinafter also referred to as "Mincle") is known to enhance the Th1 cellular immunity (Non Patent Literature 1) and is considered to be effective in protecting against infections caused by intracellular parasites. Therefore, in order to search for a new compound with an immunostimulatory action, attempts have been conventionally made to create a compound that efficiently activates Mincle (Non Patent Literature 2). However, satisfactory results have not necessarily been achieved, and the creation of a new compound that efficiently activates Mincle has been awaited.

### [Citation List]

### [Non Patent Literature]

[Non Patent Literature 1]
   Schoenen et al, J. Immunol., 2010, 184:2756
[Non Patent Literature 2]
   Braganza et al, Front. Immunol., 2018, 8:1940
[Non Patent Literature 3]
   Arbues et al, Front. Cell. Infect. Microbiol., 2014, 4:173

### [Summary of Invention]

### [Technical Problem]

In order to solve the above-mentioned problems, the present invention aims to provide a new compound that efficiently activates Mincle (Mincle ligand).

### [Solution to Problem]

The present inventors took note of the fact that the immune response to mycobacteria is initiated by pattern recognition receptors (PRRs) sensing lipids in the mycobacterial cell wall, investigated the involvement of lipids that constitute the cell wall of Mycobacterium leprae, the causative bacterium of Hansen's disease (Non Patent Literature 3), in immune responses, and found for the first time that phenolic glycolipid-III (PGL-III), whose function was conventionally unknown, is a useful Mincle ligand. Based on this new finding, the present inventors further conducted intensive studies and completed the present invention.

In the present invention, the following are disclosed as specific examples of embodiments of the present invention. However, the present invention is not limited to these.

[1] A glycolipid derivative (I) represented by the following formula (I): 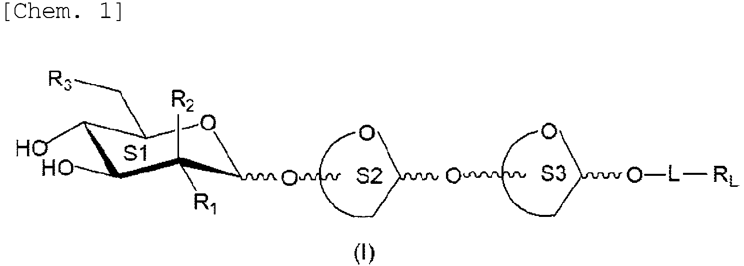
wherein one of R₁ and R₂ is a hydroxy group or a C₁₋₆ alkoxy group, and the other is a hydrogen atom,
R₃ is a hydrogen atom, a hydroxy group, or a C₁₋₆ alkoxy group, 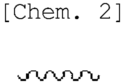
is a single bond (steric configuration is unspecified),
ring S2 and ring S3 are each independently an optionally substituted pyranose or an optionally substituted deoxypyranose, L is a C₆₋₁₄ arylene group, and
R_{L} is an optionally substituted C₃₋₄₀ hydrocarbon group,
or a salt thereof.
(provided that PGL-I, PGL-II, and PGL-III (Rx and Ry of each compound are as shown in Table 1) represented by the following formula (II): 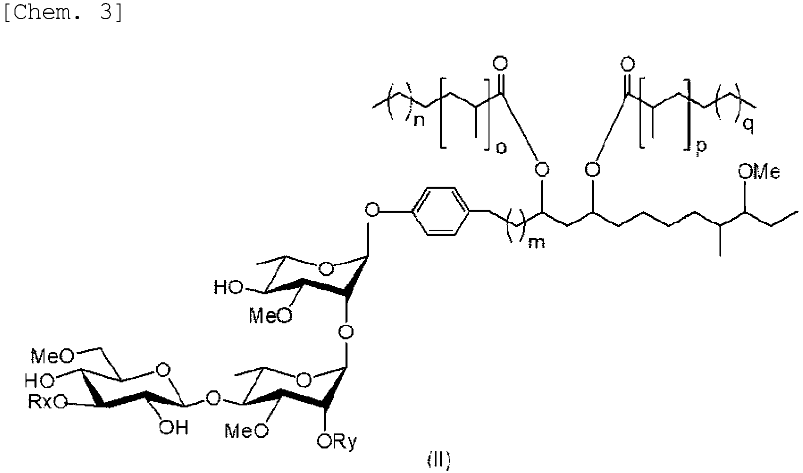
wherein m is an integer of 13 to 21, n is an integer of 15 to 17, o is an integer of 3 to 5, p is an integer of 3 to 5, and q is an integer of 15 to 17
are excluded from the glycolipid derivative (I)

**[Table 1]**

| glycolipid derivative | Rx | Ry |
|---|---|---|
| PGL-I | methyl group | methyl group |
| PGL-II | methyl group | hydrogen atom |
| PGL-III | hydrogen atom | methyl group |

[2] The glycolipid derivative (I) of the above-mentioned [1], wherein ring S2 and ring S3 are each independently a pyranose optionally substituted by a C₁₋₆ alkyl group, or a deoxypyranose optionally substituted by a C₁₋₆ alkyl group, or a salt thereof.
[3] The glycolipid derivative (I) of the above-mentioned [1] or [2], wherein ring S2 and ring S3 are each independently glucopyranose, mannopyranose, 6-deoxyglucopyranose, or 6-deoxymannopyranose (rhamnopyranose), each of which is optionally substituted by 1 to 3 C₁₋₆ alkyl groups, preferably ring S2 and ring S3 are each independently 6-deoxymannopyranose (rhamnopyranose) optionally substituted by 1 to 3 C₁₋₆ alkyl groups, or a salt thereof.
[4] The glycolipid derivative (I) of any of the above-mentioned [1] to [3], wherein the terminal sugar represented by ring S1 and the sugar represented by ring S2 are linked by a 1,4 glycosidic bond when viewed from ring S1, or a salt thereof.
[5] The glycolipid derivative (I) of any of the above-mentioned [1] to [4], wherein the sugar represented by ring S2 and the sugar represented by ring S3 are linked by a 1,2 or 1,4 glycosidic bond when viewed from ring S2, or a salt thereof.
[6] The glycolipid derivative (I) of any of the above-mentioned [1] to [5], which is a derivative represented by the following formula (Ia): 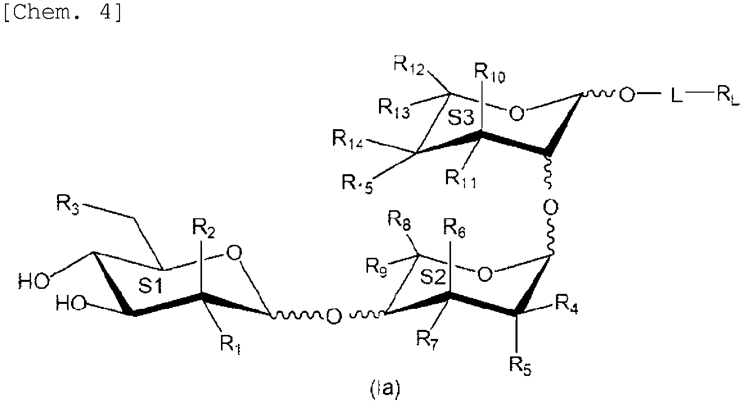 wherein
R₁, R₂, R₃, L, RL, and 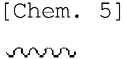
are each the same as the symbols in the glycolipid derivative (I) of the above-mentioned [1],
one of R₄ and R₅ is a hydroxy group or a C₁₋₆ alkoxy group, and the other is a hydrogen atom,
one of R₆ and R₇ is a hydroxy group or a C₁₋₆ alkoxy group, and the other is a hydrogen atom,
one of R₈ and R₉ is a group represented by the formula: CH₂-Ra wherein Ra is a hydrogen atom, a hydroxy group, or a C₁₋₆ alkoxy group, and the other is a hydrogen atom,
one of R₁₀ and R₁₁ is a hydroxy group or a C₁₋₆ alkoxy group, and the other is a hydrogen atom,
one of R₁₂ and R₁₃ is a group represented by the formula: CH₂-Ra wherein Ra is a hydrogen atom, a hydroxy group, or a C₁₋₆ alkoxy group, and the other is a hydrogen atom,
one of R₁₄ and R₁₅ is a hydroxy group or a C₁₋₆ alkoxy group, and the other is a hydrogen atom,
or a salt thereof.

[7] The glycolipid derivative (I) of any of the above-mentioned [1] to [6], wherein the terminal sugar represented by ring S1 and the sugar represented by ring S2 are linked by a β1,4 glycosidic bond when viewed from ring S1, or a salt thereof.
[8] The glycolipid derivative (I) of any of the above-mentioned [1] to [7], wherein one of R₁ and R₂ is a hydroxy group, and the other is a hydrogen atom, or a salt thereof.
[9] The glycolipid derivative (I) of any of the above-mentioned [1] to [8], wherein R₁ is a hydroxy group and R₂ is a hydrogen atom, or a salt thereof.
[10] The glycolipid derivative (I) of any of the above-mentioned [1] to [9], wherein R₃ is a hydroxy group or a C₁₋₆ alkoxy group, preferably a C₁₋₆ alkoxy group, or a salt thereof.
[11] The glycolipid derivative (I) of any of the above-mentioned [6] to [10], wherein one of R₄ and R₅ is a C₁₋₆ alkoxy group and the other is a hydrogen atom,
one of R₆ and R₇ is a C₁₋₆ alkoxy group and the other is a hydrogen atom, and
one of R₈ and R₉ is a group represented by the formula: CH₂-Ra wherein Ra is a hydrogen atom, and the other is a hydrogen atom, or a salt thereof.

[12] The glycolipid derivative (I) of any of the above-mentioned [6] to [11], wherein R₄ is a hydrogen atom and R₅ is a C₁₋₆ alkoxy group,
R₆ is a hydrogen atom and R₇ is a C₁₋₆ alkoxy group, and
R₈ is a hydrogen atom and R₉ is a group represented by the formula: CH₂-Ra wherein Ra is a hydrogen atom,
   or a salt thereof.

[13] The glycolipid derivative (I) of any of the above-mentioned [6] to [12], wherein one of R₁₀ and R₁₁ is a C₁₋₆ alkoxy group and the other is a hydrogen atom,
one of R₁₂ and R₁₃ is a group represented by the formula: CH₂-Ra wherein Ra is a hydrogen atom, and the other is a hydrogen atom, and
one of R₁₄ and R₁₅ is a hydroxy group and the other is a hydrogen atom,
or a salt thereof.

[14] The glycolipid derivative (I) of any of the above-mentioned [6] to [13], wherein R₁₀ is a hydrogen atom, R₁₁ is a C₁₋₆ alkoxy group,
R₁₂ is a hydrogen atom, R₁₃ is a group represented by the formula: CH₂-Ra wherein Ra is a hydrogen atom,
R₁₄ is a hydroxy group, and R₁₅ is a hydrogen atom,
or a salt thereof.

[15] The glycolipid derivative (I) of any of the above-mentioned [1] to [14], wherein L is a phenylene group, or a salt thereof.
[16] The glycolipid derivative (I) of any of the above-mentioned [1] to [15], wherein, in R_{L}, the hydrocarbon group in the optionally substituted C₃₋₄₀ hydrocarbon group is a C₆₋₄₀ hydrocarbon group (preferably a C₆₋₃₅ hydrocarbon group, more preferably a C₆₋₃₅ alkyl group), or a salt thereof.
[17] The glycolipid derivative (I) of any of the above-mentioned [1] to [16], wherein, in R_{L}, the optionally substituted C₃₋₄₀ hydrocarbon group has the same or different 1 to 5 substituents selected from
1) a hydroxy group,
2) an oxo group,
3) an optionally substituted C₃₋₄₀ hydrocarbon group (preferably a C₃₋₄₀ hydrocarbon group optionally substituted by the same or different 1 to 5 substituents selected from a hydroxy group, an oxo group, and a C₁₋₆ alkoxy group),
4) a C₁₋₆ alkoxy group,
5) an optionally substituted C₃₋₄₀ hydrocarbon-carbonyloxy group (preferably a C₆₋₄₀ hydrocarbon-carbonyloxy group optionally substituted by the same or different 1 to 5 substituents selected from a hydroxy group, an oxo group, and a C₁₋₆ alkoxy group),
6) a group containing an epitope sequence,
7) an amino group or an acylamino group (preferably an amino group or an optionally substituted C₃₋₄₀ hydrocarbon-carbonylamino group),
8) a thiol group, and
9) a halogen atom,
more preferably the same or different 1 to 5 substituents selected from
a) a hydroxy group,
b) an oxo group,
c) a C₁₋₆ alkoxy group, and
d) an optionally substituted C₃₋₄₀ hydrocarbon-carbonyloxy group (preferably a C₆₋₄₀ hydrocarbon-carbonyloxy group optionally substituted by the same or different 1 to 5 substituents selected from a hydroxy group, an oxo group, and a C₁₋₆ alkoxy group), wherein the C₃₋₄₀ hydrocarbon group optionally has 1 to 5 ring structure moieties in the molecule,
or a salt thereof.
[18] A pharmaceutical composition comprising a glycolipid derivative (I') represented by the following formula (I'): 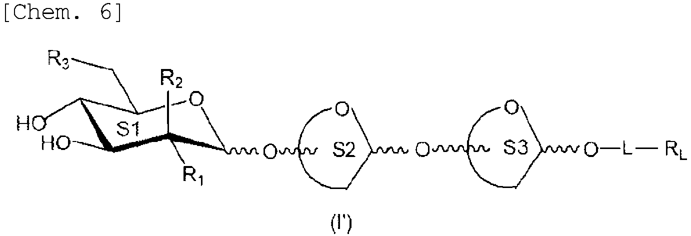 wherein each symbol has the same meaning as the symbol in the glycolipid derivative (I) described in the above-mentioned [1], or a salt thereof as an active ingredient (provided that PGL-I and PGL-II (Rx and Ry of each compound are as shown in Table 2) represented by the following formula (II): 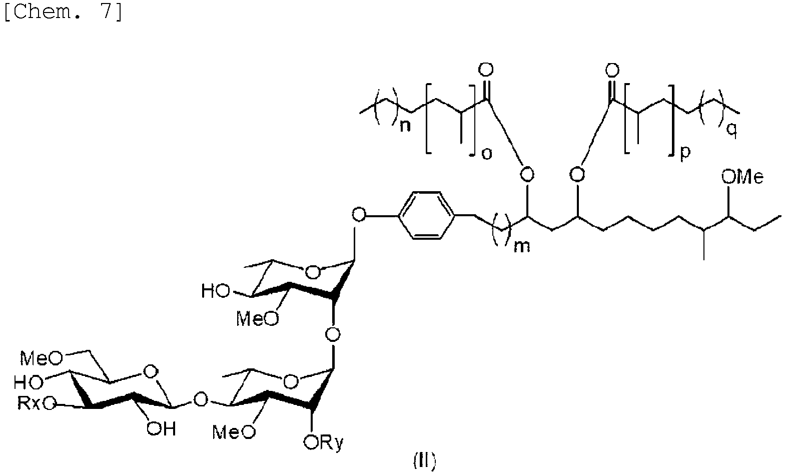
wherein m is an integer of 13 to 21, n is an integer of 15 to 17, o is an integer of 3 to 5, p is an integer of 3 to 5, and q is an integer of 15 to 17
are excluded from the glycolipid derivative (I')

**[Table 2]**

| glycolipid derivative | Rx | Ry |
|---|---|---|
| PGL-I | methyl group | methyl group |
| PGL-II | methyl group | hydrogen atom |

[19] The pharmaceutical composition of the above-mentioned [18], which is a Mincle activator.
[20] The pharmaceutical composition of the above-mentioned [18] or [19], which is a TNF production enhancer.
[21] The pharmaceutical composition of any of the above-mentioned [18] to [20], which is an anticancer agent.
[22] The pharmaceutical composition of the above-mentioned [18] or [19], which is an INF-γ production enhancer.
[23] The pharmaceutical composition of the above-mentioned [18], [19] or [22], which is an agent for the prophylaxis and/or treatment of a bacterial infection, a viral infection, and/or an allergic disease.
[24] The pharmaceutical composition of the above-mentioned [18] or [19], which is an immunostimulant.
[25] The pharmaceutical composition of the above-mentioned [18], [19] or [24], which is a vaccine adjuvant.
[26] The pharmaceutical composition of the above-mentioned [25], further comprising an antigen.
[27] The pharmaceutical composition of any of the above-mentioned [18] to [26], wherein the glycolipid derivative (I') or a salt thereof is a PGL-III compound represented by 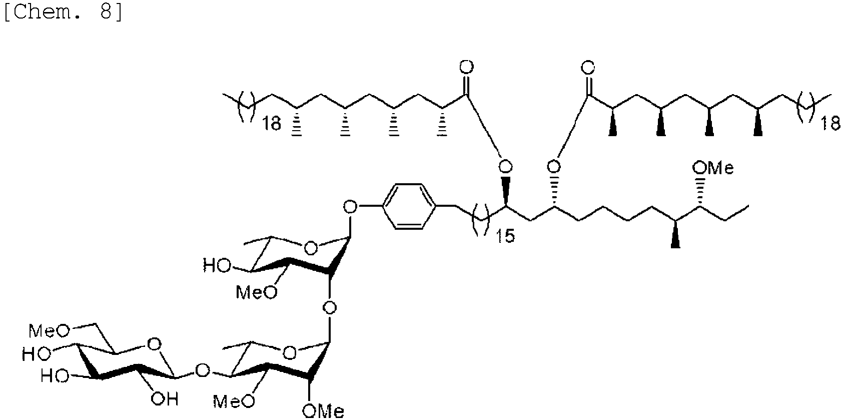 or a salt thereof.
[28] A method for the prophylaxis and/or treatment of cancer, a bacterial infection, a viral infection, and/or an allergic disease, comprising administering an effective amount of the glycolipid derivative (I') defined in the above-mentioned [18] or a salt thereof to a mammal in need of the administration.
[29] A method for stimulating immunity, comprising administering an effective amount of the glycolipid derivative (I') defined in the above-mentioned [18] or a salt thereof to a mammal in need of the administration.
[30] The glycolipid derivative (I') defined in the above-mentioned [18] or a salt thereof for use in the prophylaxis and/or treatment of cancer, a bacterial infection, a viral infection, and/or an allergic disease.
[31] The glycolipid derivative (I') defined in the above-mentioned [18] or a salt thereof for use in stimulating immunity.
[32] Use of the glycolipid derivative (I') defined in the above-mentioned [18] or a salt thereof for the production of a medicament for the prophylaxis and/or treatment of cancer, a bacterial infection, a viral infection, and/or an allergic disease.
[33] Use of the glycolipid derivative (I') defined in the above-mentioned [18] or a salt thereof for the production of an immunostimulant.

### [Advantageous Effects of Invention]

The present invention provides, as embodiments thereof, a glycolipid derivative having a Mincle activating action, as well as use of the glycolipid derivative, including use thereof as a vaccine adjuvant.

### [Brief Description of Drawings]

[Fig. 1]

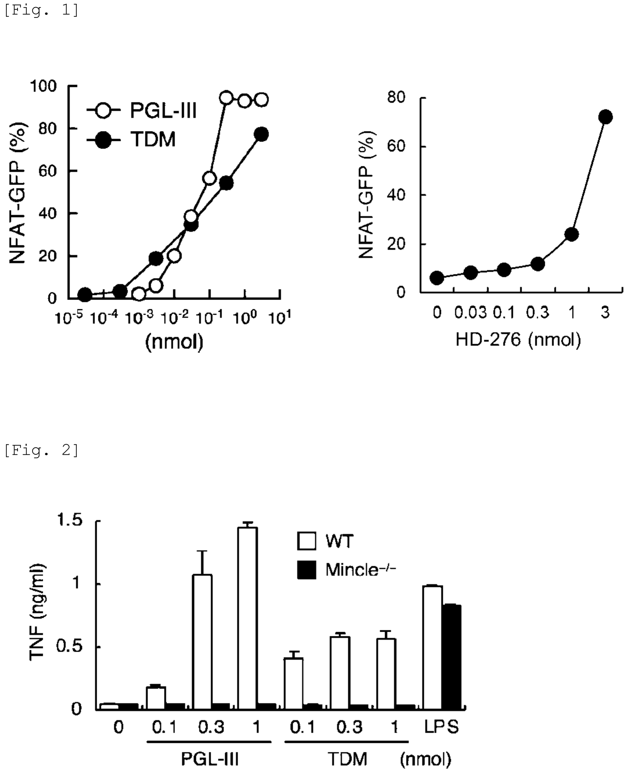

   Fig. 1 shows the evaluation results of Mincle ligand activity of glycolipid derivatives in Experimental Example 1.
[Fig. 2]
   Fig. 2 shows the evaluation results of macrophage activation by glycolipid derivative in Experimental Example 2.
[Fig. 3]

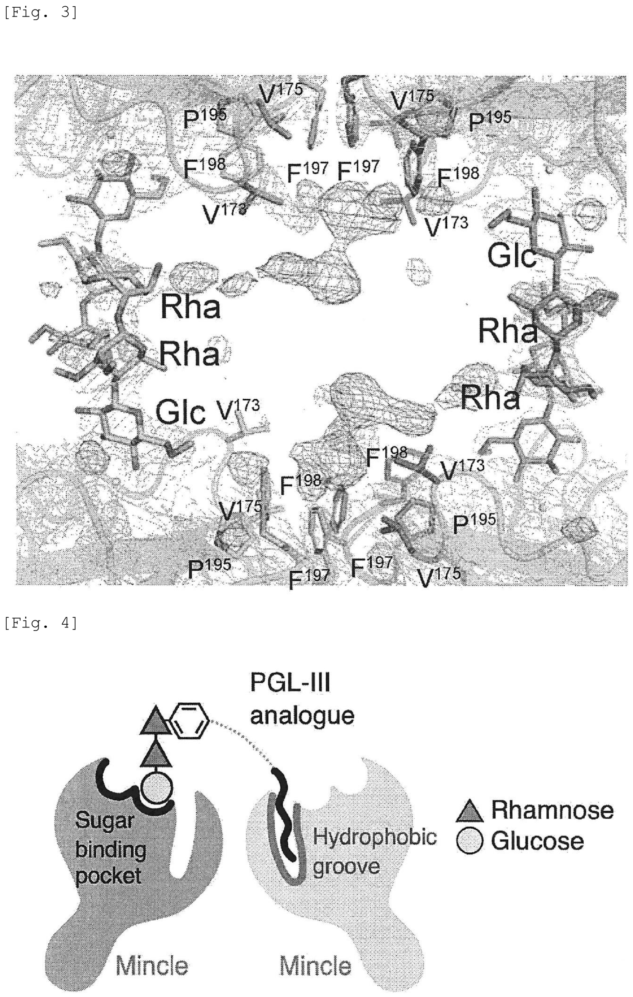

   Fig. 3 shows the crystal structure analysis results (electron density map) of Mincle-glycolipid derivative complex in Experimental Example 3.
[Fig. 4]
   Fig. 4 shows the crystal structure analysis results (binding mode schematic diagram) of Mincle-glycolipid derivative complex in Experimental Example 3.
[Fig. 5]

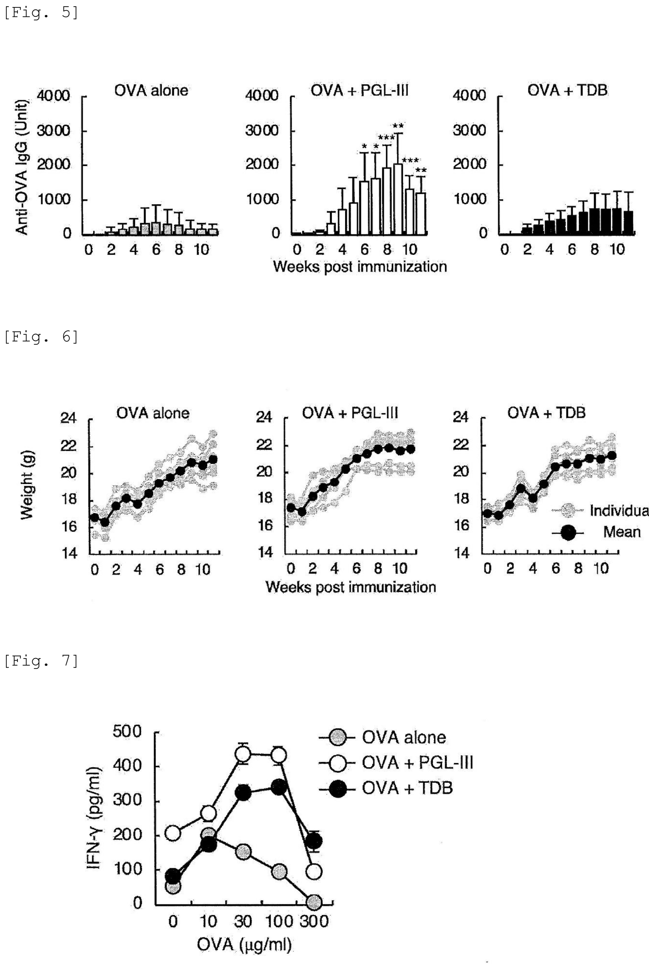

   Fig. 5 shows the evaluation results of the antigen-specific IgG production-enhancing effect of glycolipid derivatives in Experimental Example 4(1).
[Fig. 6]
   Fig. 6 shows the evaluation results of the influence of glycolipid derivatives on the body weight in Experimental Example 4(2).
[Fig. 7]
   Fig. 7 shows the evaluation results of the cellular immunity-enhancing effect of glycolipid derivatives in Experimental Example 5.

### [Description of Embodiments]

The present invention is described in detail below. Unless otherwise specified, all technical and scientific terms used in the present specification have the same meaning as generally understood by one of ordinary skill in the art to which the present invention belongs. While any methods and materials similar or equivalent to those described in the present specification can be used in the practice or testing of the present invention, preferred methods and materials are described below. All publications and patents mentioned herein are incorporated by reference for the purpose of describing and disclosing, for example, the constructs and methodologies described in the publications that may be used in connection with the described invention.

### [Glycolipid derivative (I) of the present invention]

The present invention provides, as one embodiment thereof, "a glycolipid derivative (I) represented by the following formula (I): 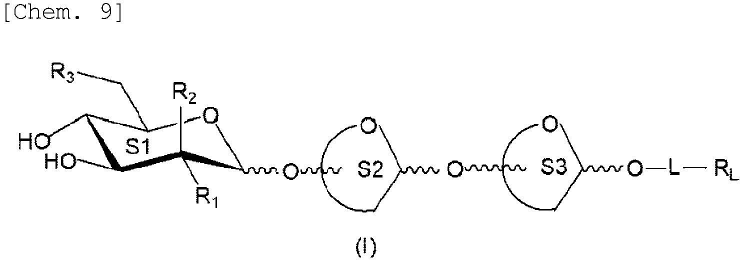
wherein one of R₁ and R₂ is a hydroxy group or a C₁₋₆ alkoxy group, and the other is a hydrogen atom,
R₃ is a hydrogen atom, a hydroxy group, or a C₁₋₆ alkoxy group, 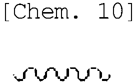
is a single bond (steric configuration is unspecified),
ring S2 and ring S3 are each independently an optionally substituted pyranose or an optionally substituted deoxypyranose, L is a C₆₋₁₄ arylene group, and
R_{L} is an optionally substituted C₃₋₄₀ hydrocarbon group,
or a salt thereof.
(provided that the aforementioned PGL-I, PGL-II, and PGL-III are excluded from the above-mentioned glycolipid derivative (I))".

The following explains each group in the above-mentioned glycolipid derivative (I).

In the present specification, the notation "C_{a-b}" (e.g., C₁₋₆) indicates that the number of carbon atoms constituting the group is a to b (e.g., 1 to 6).

In the present specification, the notation "optionally substituted" indicates that the group may be substituted at any substitutable position in the target to be substituted.

In the present specification, examples of the "halogen atom" include fluorine, chlorine, bromine, and iodine.

In the present specification, examples of the "C₁₋₆ alkyl (group)" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

In the present specification, examples of the "C₁₋₆ alkoxy (group)" include methoxy, ethoxy, propoxy, isopropoxy, butoxy, isobutoxy, sec-butoxy, tert-butoxy, pentyloxy, and hexyloxy.

In the present specification, the "pyranose" refers to an aldohexose (six-carbon sugar) carbohydrate that forms a six-membered ring with five carbons and one oxygen at the vertices. Examples include allopyranose, altropyranose, glucopyranose, mannopyranose, gulopyranose, idopyranose, galactopyranose, and talopyranose. In practicing the present invention, either the D-form or L-form of "pyranose" can be used. Furthermore, the "pyranose" to be used may be natural or unnatural.

The hydrogen atoms of the hydroxy groups of the "pyranose" may be substituted, and suitable substituents include C₁₋₆ alkyl groups (e.g., methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, etc.). The number of substituted hydroxy groups is preferably 1 to 3.

In the present specification, the "deoxypyranose" refers to a pyranose having a structure in which one or more hydroxy groups of the above-mentioned "pyranose (aldohexose)" have been substituted by hydrogen atoms, and examples include 2- (or 3- or 4- or 5- or 6-) deoxyallopyranose, 2- (or 3- or 4- or 5- or 6-) deoxyaltropyranose, 2- (or 3- or 4- or 5- or 6-) deoxyglucopyranose, 2- (or 3- or 4- or 5- or 6-) deoxymannopyranose, 2- (or 3- or 4- or 5- or 6-) deoxygulopyranose, 2- (or 3- or 4- or 5- or 6-) deoxyidopyranose, 2- (or 3- or 4- or 5- or 6-) deoxygalactopyranose, and 2- (or 3- or 4- or 5- or 6-) deoxytalopyranose. Without limitation, preferred examples include 2- (or 3-, 4-, 5-, or 6-) deoxyglucopyranose and 2- (or 3-, 4-, 5-, or 6-) deoxymannopyranose, more preferred examples include 6-deoxypyranose, and further preferred examples include 6-deoxyglucopyranose and 6-deoxymannopyranose (rhamnopyranose).

The hydrogen atom of the hydroxy group of the "deoxypyranose" may be substituted, and preferred examples include C₁₋₆ alkyl groups (e.g., methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, etc.). The number of substituted hydroxy groups is preferably 1 to 3.

In the present specification, the "C₆₋₁₄ arylene group" refers to a divalent group derived from C₆₋₁₄ arene (e.g., benzene, naphthalene, etc.), and examples include 1,2-, 1,3-, or 1,4-phenylene, 1,2-, 1,3-, 2,3-1,4-, 1,5-, 1,6-1,7-, and 1,8-naphthylene.

In the present specification, the "C₃₋₄₀ hydrocarbon group" refers to a saturated or unsaturated, linear or branched hydrocarbon group having 3 to 40 carbon atoms, and specific examples include propyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, 2-hexenyl, 1-hexynyl, heptyl, 1-methylhexyl, 6-heptynyl, octyl, 2-methylheptyl, 2-octen-7-ynyl, nonyl, 3-methyloctyl, decyl, 4-methylnonyl, undecyl, 5-methyldecyl, dodecyl, 6-methylundecyl, tridecyl, 7-methylundecyl, tetradecyl, 8-methyltridecyl, pentadecyl, 9-methyltetradecyl, hexadecyl, 10-methylpentadecyl, 12-hexadecenyl, 1-hexadecynyl, heptadecyl, 11-methylhexadecyl, 16-heptadecynyl, octadecyl, 12-methylhexadecyl, 12-octadecen-17-ynyl, nonadecyl, 13-methyloctadecyl, icosyl, 14-methylnonadecyl, henicosyl, 15-methylicosyl, docosyl, 16-ethylicosyl, tricosyl, 10,17-dimethylhenicosyl, tetracosyl, 10,18-dimethylhenicosyl, pentacosyl, 19-ethyltricosyl, hexacosyl, 22-hexacosenyl, 1-hexacosinyl, 10,20-dimethyltetracosyl, heptacosyl, 21-methylhexacosyl, 26-heptacosinyl, octacosyl, 10,21-dimethylhexacosyl, 22-hexacosen-27-ynyl, nonacosyl, 10-methyl-15-ethyl-22-propyltriacosyl, triacontyl, 24-methylnonacosyl, hentriacontyl, 25-methyltriacontyl, 1,3,5,7-tetramethylheptacosyl, dotriacontyl, 26-ethyltriacontyl, tritriacontyl, 20,27-dimethylhentriacontyl, tetratriacontyl, 20,28-dimethyldotriacontyl, pentatriacontyl, 29-methyltetracontyl, hexatriacontyl, 20,30-dimethyltetratriacontyl, 32-hexatriacontenyl, 1-hexatriacontinyl, heptatriacontyl, 31-methylhexatriacontyl, 36-heptacontinyl, octatriacontyl, 30,31-dimethylhexatriacontyl, 32-octatriaconten-37-ynyl, nonatriacontyl, 20-methyl-25-ethyl-32-propyltritriacontyl, tetracontyl, 34-methylnonatriacontyl, and the like.

As used herein, the "C₃₋₄₀ hydrocarbon group" may have 1 to 5 ring structure moieties (for example, C₃₋₆ cycloalkane moieties (e.g., cyclopropane, cyclobutane, cyclopentane, cyclohexane) and the like) in the molecule. Examples include groups represented by the following formulas: 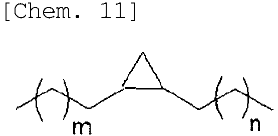 wherein m and n are each independently an integer, and the sum of m and n is 33 or less.

The "C₃₋₄₀ hydrocarbon group" is preferably a hydrocarbon group having 6 or more carbon atoms, more preferably a hydrocarbon group having 6 to 35 carbon atoms. More specifically, examples include saturated or unsaturated hydrocarbon groups having 6 to 10, 11 to 15, 16 to 20, 21 to 25, 26 to 30, or 30 to 35 carbon atoms. Furthermore, a hydrocarbon group having 1 to 5 unsaturated bonds in the molecule, or a saturated hydrocarbon group (alkyl group), each of which may have 1 to 5 ring structures in the molecule, is preferred.

Examples of the substituent with which the "C₃₋₄₀ hydrocarbon group" is optionally substituted include
1) a hydroxy group,
2) an oxo group,
3) an optionally substituted C₃₋₄₀ hydrocarbon group,
4) a C₁₋₆ alkoxy group,
5) an optionally substituted C₃₋₄₀ hydrocarbon-carbonyloxy group,
6) a group containing an epitope sequence,
7) an amino group or an acylamino group (preferably, an amino group or an optionally substituted C₃₋₄₀ hydrocarbon-carbonylamino group),
8) a thiol group, and
9) a halogen atom.

Among the above-mentioned substituents, more preferred are
1) a hydroxy group,
2) a C₁₋₆ alkoxy group, and
3) an optionally substituted C₃₋₄₀ hydrocarbon-carbonyloxy group.

As to the "C₃₋₄₀ hydrocarbon" moiety in the above-mentioned "optionally substituted C₃₋₄₀ hydrocarbon group", "optionally substituted C₃₋₄₀ hydrocarbon-carbonyloxy group", and "optionally substituted C₃₋₄₀ hydrocarbon-carbonylamino group", the explanation for the aforementioned "C₃₋₄₀ hydrocarbon group" can be referred to, and examples of substituents include a hydroxy group, an oxo group, a C₁₋₆ alkoxy group, and the like.

The "group containing an epitope sequence" includes a group containing an epitope sequence determined according to the target antigen (e.g., peptide group containing an epitope sequence), and may also be the epitope sequence itself. Epitope sequences include T cell epitopes and B cell epitopes. Those skilled in the art can select the "group containing epitope sequence" according to the purpose and link same to the "C₃₋₄₀ hydrocarbon group" as appropriate by referring to methods known in the art (e.g., C.C. Hanna et al, Chem. Commun., 2022, 58, 6890-6893).

Preferred embodiments of the groups represented by each symbol in glycolipid derivative (I) are described below.

Ring S2 and ring S3 are as defined above, and ring S2 and ring S3 may each be any sugar independently selected from an optionally substituted pyranose and an optionally substituted deoxypyranose. For example, ring S2 and ring S3 may each be an optionally substituted pyranose, or an optionally substituted deoxypyranose, or one of ring S2 and ring S3 may be an optionally substituted pyranose and the other may be an optionally substituted deoxypyranose. Furthermore, the sugar represented by ring S2 (hereinafter also simply referred to as "ring S2") and the sugar represented by ring S3 (hereinafter also simply referred to as "ring S3") may both be in D-form or both in L-form. One of ring S2 and ring S3 may be in D-form, and the other may be in L-form.

Those skilled in the art can practice the present invention by appropriately selecting ring S2 and ring S3. Specific examples include, but are not limited to, the following combinations.
(1) Ring S2 and ring S3 are each independently an optionally substituted pyranose or an optionally substituted deoxypyranose.
(2) Ring S2 and ring S3 are each independently a pyranose (preferably glucopyranose or mannopyranose) optionally substituted by a C₁₋₆ alkyl group or a deoxypyranose (preferably 6-deoxyglucopyranose or 6-deoxymannopyranose (rhamnopyranose)) optionally substituted by a C₁₋₆ alkyl group.
(3) Ring S2 and ring S3 are each independently a deoxypyranose optionally substituted by 1 to 3 C₁₋₆ alkyl groups.
(4) Ring S2 and ring S3 are each independently a deoxymannopyranose optionally substituted by 1 to 3 C₁₋₆ alkyl groups.
(5) Ring S2 and ring S3 are each independently a 6-deoxymannopyranose (rhamnopyranose) optionally substituted by 1 to 3 C₁₋₆ alkyl groups. 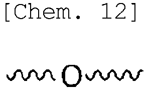
between ring S1 and ring S2, and between ring S2 and ring S3 indicates a glycosidic bond linking the two sugars.

The type of glycosidic bond that links the terminal sugar represented by ring S1 (hereinafter also simply referred to as "ring S1") to the sugar represented by ring S2 is not particularly limited, and it may be any of a 1,2 glycosidic bond, a 1,3 glycosidic bond, a 1,4 glycosidic bond, and a 1,6 glycosidic bond, preferably a 1,4 glycosidic bond, when viewed from ring S1.

The configuration (a-configuration or β-configuration) of each sugar in the glycosidic bond is not particularly limited, and a β-configuration is preferred in ring S1. Ring S2 may have either the α-configuration or the β-configuration.

The type of glycosidic bond that links ring S2 and ring S3 is not particularly limited, and it may be any of a 1,2 glycosidic bond, a 1,3 glycosidic bond, a 1,4 glycosidic bond, and a 1,6 glycosidic bond, preferably a 1,2 glycosidic bond or a 1,4 glycosidic bond, more preferably a 1,2 glycosidic bond, when viewed from ring S2.

The configuration (α-configuration or β-configuration) of each sugar in the glycosidic bond is not particularly limited, and an α-configuration is preferred in ring S2. Furthermore, ring S3 may have either the α-configuration or the β-configuration.

One of the preferred bonding types for the above-mentioned ring S1, ring S2 and ring S3 is the structure represented by the formula (Ia) described in [6] in the aforementioned [Solution to Problem], and the bonding type represented by the structure of the following formula (Ib) is more preferred. For preferred embodiments of each group in the structure of the formula (Ib), reference can be made to the embodiments described in [8] to [17] for the derivatives of the formula (Ia).

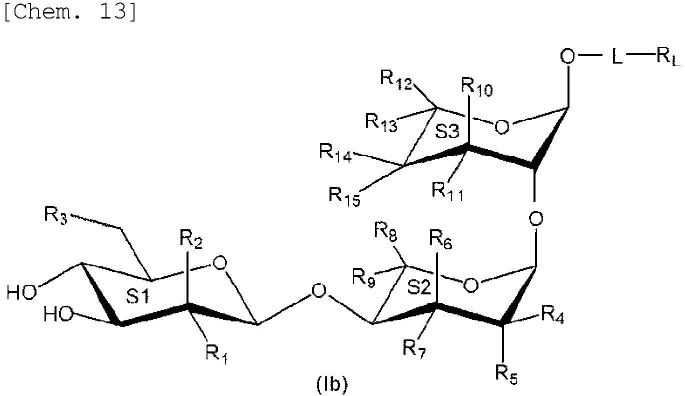

R₁ and R₂ are as defined above, but it is preferable that one of them is a hydroxy group, and the other is a hydrogen atom, and it is more preferable that R₁ is a hydroxy group and R₂ is a hydrogen atom.

R₃ is as defined above, and is preferably a hydroxy group or a C₁₋₆ alkoxy group, more preferably a C₁₋₆ alkoxy group, particularly preferably a methoxy group.

R₄ and R₅ are as defined above, but it is preferable that one of them is a C₁₋₆ alkoxy group and the other is a hydrogen atom, and it is more preferable that R₄ is a hydrogen atom and R₅ is a C₁₋₆ alkoxy group.

R₆ and R₇ are as defined above, but it is preferable that one of them is a C₁₋₆ alkoxy group and the other is a hydrogen atom, and it is more preferable that R₆ is a hydrogen atom and R₇ is a C₁₋₆ alkoxy group.

R₈ and R₉ are as defined above, but it is preferable that one of them is a group represented by the formula: CH₂-Ra wherein Ra is a hydrogen atom, and the other is a hydrogen atom, and it is more preferable that R₈ is a hydrogen atom and R₉ is a group represented by the formula: CH₂-Ra wherein Ra is a hydrogen atom.

R₁₀ and R₁₁ are as defined above, but it is preferable that one of them is a C₁₋₆ alkoxy group and the other is a hydrogen atom, and it is more preferable that R₁₀ is a hydrogen atom and R₁₁ is a C₁₋₆ alkoxy group.

R₁₂ and R₁₃ are as defined above, but it is preferable that one of them is a group represented by the formula: CH₂-Ra wherein Ra is a hydrogen atom, and the other is a hydrogen atom, and it is more preferable that R₁₂ is a hydrogen atom and R₁₃ is a group represented by the formula: CH₂-Ra wherein Ra is a hydrogen atom.

R₁₄ and R₁₅ are as defined above, but it is preferable that one of them is a hydroxy group and the other is a hydrogen atom, and it is more preferable that R₁₄ is a hydroxy group and R₁₅ is a hydrogen atom.

L is as defined above, and is preferably a phenylene group, more preferably a 1,4-phenylene group.

R_{L} is as defined above, and is preferably
an optionally substituted C₆₋₄₀ hydrocarbon group,
more preferably
a C₆₋₄₀ hydrocarbon group optionally substituted by the same or different 1 to 5 substituents selected from 1) a hydroxy group, 2) an oxo group, 3) an optionally substituted C₃₋₄₀ hydrocarbon group, 4) a C₁₋₆ alkoxy group, 5) an optionally substituted C₃₋₄₀ hydrocarbon-carbonyloxy group, 6) a group containing an epitope sequence, 7) an amino group or an optionally substituted C₃₋₄₀ hydrocarbon-carbonylamino group, 8) a thiol group, and 9) a halogen atom,
further preferably
a C₆₋₄₀ hydrocarbon group optionally substituted by the same or different 1 to 5 substituents selected from 1) a hydroxy group, 2) an oxo group, 3) a C₃₋₄₀ hydrocarbon group optionally substituted by the same or different 1 to 5 substituents selected from a hydroxy group, an oxo group, and a C₁₋₆ alkoxy group, 4) a C₁₋₆ alkoxy group, 5) a C₃₋₄₀ hydrocarbon-carbonyloxy group optionally substituted by the same or different 1 to 5 substituents selected from a hydroxy group, an oxo group, and a C₁₋₆ alkoxy group, 6) a group containing an epitope sequence, 7) an amino group, or a C₃₋₄₀ hydrocarbon-carbonylamino group optionally substituted by the same or different 1 to 5 substituents selected from a hydroxy group, an oxo group, and a C₁₋₆ alkoxy group, 8) a thiol group, and 9) a halogen atom, particularly preferably
a C₆₋₄₀ alkyl group optionally substituted by the same or different 1 to 5 substituents selected from 1) a hydroxy group, 2) a C₁₋₆ alkoxy group, 3) a C₃₋₄₀ hydrocarbon-carbonyloxy group optionally substituted by the same or different 1 to 5 substituents selected from a hydroxy group, an oxo group, and a C₁₋₆ alkoxy group.

In the above, the C₆₋₄₀ hydrocarbon group and the C₆₋₄₀ alkyl group are more preferably a C₆₋₃₅ hydrocarbon group and a C₆₋₃₅ alkyl group, respectively. In addition, the C₆₋₄₀ hydrocarbon group and the C₆₋₄₀ alkyl group may each have 1 to 5 ring structure moieties in the molecule.

In practicing the present invention, glycolipid derivative (I) can be used in either a free form or a salt form (preferably a pharmaceutically acceptable salt thereof). Those skilled in the art can practice the present invention by appropriately selecting from the both forms, taking into account the properties of the individual glycolipid derivative (I) to be used.

Examples of the pharmaceutically acceptable salt include salts with inorganic acids such as hydrochloride, hydrobromide, sulfate, and phosphate; salts with organic acids such as acetate, fumarate, oxalate, citrate, methanesulfonate, benzenesulfonate, tosylate, and maleate; salts with bases such as alkali metal salts such as sodium salt and potassium salt, and alkaline earth metal salts such as calcium salt; and salts with amino acids such as glycine salt, lysine salt, arginine salt, ornithine salt, glutamic acid salt, and aspartic acid salt.

The glycolipid derivative (I) or a salt thereof of the present invention has been described in detail above. Based on the results of "Analysis of crystal structure of Mincle-ligand complex" described in Experimental Example 3 below, guidelines for specifically designing glycolipid derivative (I) are described.

From the structural analysis, it can be seen that in glycolipid derivative (I), the sugar chain portion (oligosaccharide portion consisting of ring S1-ring S2-ring S3) and the lipid portion (R_{L} portion) each interact with Mincle, inducing efficient polymerization of Mincle. By observation in more detail, as can be seen from Fig. 4, only the terminal sugar represented by S1 (S1 sugar) binds to Mincle rather than binding of each of the three sugars along the surface of Mincle, and the sugars represented by S2 and S3 (S2 sugar, S3 sugar) do not bind to Mincle but are arranged at an angle close to perpendicular to the surface of Mincle. Therefore, while it can be seen that there is a relatively large degree of freedom in selecting the S2 sugar and S3 sugar when designing glycolipid derivative (I), it is preferable to design the S2 sugar and S3 sugar to be arranged as described above (for example, using linking mode β between the S1 sugar and S2 sugar).

On the other hand, by observation of the lipid portion (R_{L} portion), as can be seen from Fig. 4, a hydrocarbon chain with an appropriate chain length is arranged to protrude from the periphery of Mincle instead of along the surface of Mincle to which it is bound via sugar, and the hydrocarbon chain is bound to the hydrophobic groove of Mincle. Therefore, it is understood to be preferable to design the lipid portion (R_{L} portion) to satisfy such binding mode (for example, setting the carbon number to around 6 or more) when designing glycolipid derivative (I).

Those skilled in the art can design, produce, and use the desired glycolipid derivative (I) by referring to the above guidelines.

### [Production method of glycolipid derivative (I) of the present invention]

The production method of glycolipid derivative (I) or a salt thereof is described below.

Glycolipid derivative (I) or a salt thereof can be produced, for example, by the synthesis scheme shown below. 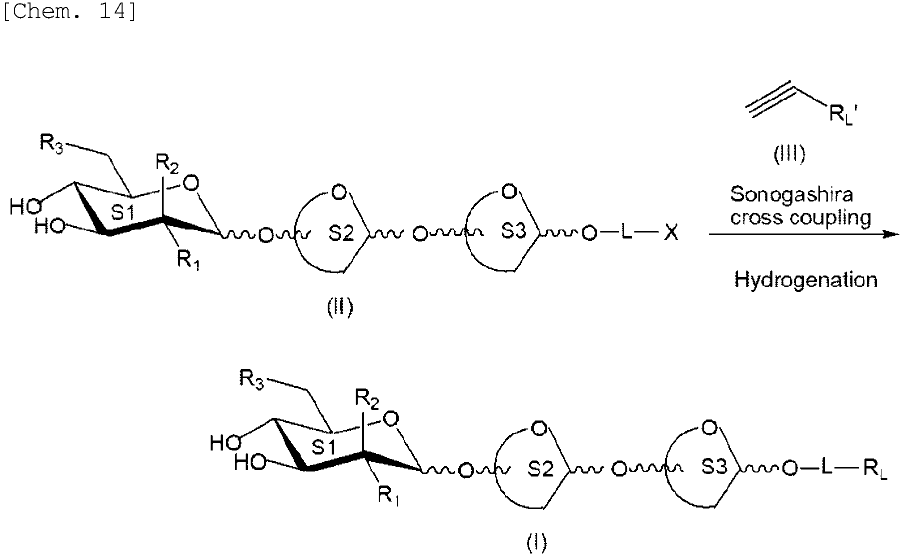 wherein, in the above-mentioned formula, R_{L}' is an optionally substituted C₃₋₃₈ hydrocarbon group, X is a halogen atom (e.g., fluorine atom, chlorine atom, bromine atom, iodine atom), and other symbols are each the same as those in glycolipid derivative (I) described in the above-mentioned [1].

Glycolipid derivative (I) or a salt thereof can be produced by the above-mentioned synthesis scheme, by subjecting raw material compound (II) or a salt thereof (hereinafter also referred to as "compound (II)") and raw material compound (III) or a salt thereof (hereinafter also referred to as "compound (III)") to a Sonogashira cross-coupling reaction, and subjecting the obtained compound or a salt thereof to a hydrogenation reaction.

For salts of each raw material compound, reference can be made to those exemplified for glycolipid derivative (I).

Each reaction in the above-mentioned synthesis scheme can be performed appropriately by a person skilled in the art by reference to the specific methods described in the Production Examples and Examples described below or methods known in the art.

Compound (II) in the above-mentioned synthesis scheme can be produced appropriately by a person skilled in the art by starting from a known compound by reference to the specific methods described in the Production Examples and Examples described below or methods known in the art, after determining the structure of the sugar moiety of the desired glycolipid derivative by selecting the sugar represented by ring S3 and the sugar represented by ring S2.

When compound (III) has a "hydroxy group" as (one of) the substituent and does not have an "optionally substituted C₃₋₄₀ hydrocarbon-carbonyloxy group" as a substituent, glycolipid derivative (I) or a salt thereof can also be produced by subjecting same to a Sonogashira cross coupling reaction with compound (II), followed by esterification with a carboxylic acid compound corresponding to the "optionally substituted C₃₋₄₀ hydrocarbon-carbonyloxy group", and then subjecting same to a hydrogenation reaction. This synthesis reaction can also be performed appropriately by a person skilled in the art by starting from a known compound by reference to the specific methods described in the Production Examples and Examples described below or methods known in the art.

### [Usefulness of glycolipid derivative (I) of the present invention]

The present invention provides, as another embodiment, "a pharmaceutical composition containing glycolipid derivative (I') represented by the following formula (I'): 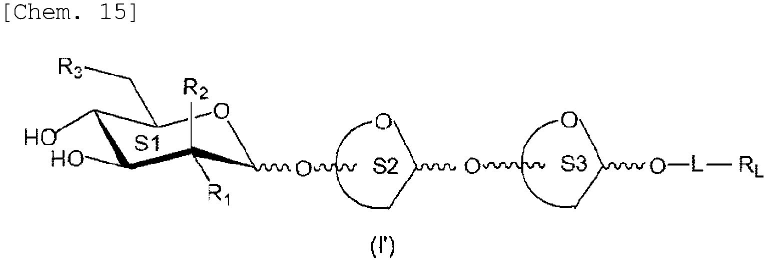 wherein each symbol is the same as that in glycolipid derivative (I) described in the above-mentioned [1], or a salt thereof as an active ingredient (provided that the aforementioned PGL-I and PGL-II are excluded from the above-mentioned glycolipid derivative (I'))".

Glycolipid derivative (I') is a compound common to the aforementioned glycolipid derivative (I) except that PGL-III are included in the scope thereof. Therefore, for the definition of each group and preferred embodiments thereof, reference can be made to the aforementioned corresponding descriptions for glycolipid derivative (I) (for example, the embodiments of "glycolipid derivative (I) or a salt thereof" described in [1] to [17] in the aforementioned [Solution to Problem]).

Since glycolipid derivative (I) is encompassed in glycolipid derivative (I'), the usefulness of glycolipid derivative (I') or a salt thereof detailed in the following also applies to glycolipid derivative (I) or a salt thereof.

For salts of glycolipid derivative (I'), reference can be made to those exemplified for glycolipid derivative (I).

### (1) Use for prophylaxis/treatment of disease

Glycolipid derivative (I) or (I') (hereinafter collectively referred to as glycolipid derivative (I')) or a salt thereof is useful as a medicament for the prophylaxis and/or treatment of disease, which contains the glycolipid derivative (I) or (I') itself as an active ingredient.

As demonstrated by the representative compounds in the Experimental Examples described later, glycolipid derivative (I') or a salt thereof is a Mincle ligand and can activate the action of Mincle. Through this activation of Mincle, the activity of the innate immune system is triggered, and, for example, a defense action against infection against intracellular parasitic pathogens is exerted. Furthermore, glycolipid derivative (I') or a salt thereof activates bone marrow-derived macrophages through Mincle activation, and affords an action to enhance TNF (tumor necrosis factor) production. Therefore, it is useful as an anticancer agent (agent for the prophylaxis and/or treatment of cancer). Target cancer is not particularly limited, and examples include cancers [e.g., colon cancer, lung cancer, mesothelioma, pancreatic cancer, pharyngeal cancer, laryngeal cancer, esophageal cancer, gastric cancer, duodenal cancer, small intestine cancer, breast cancer, ovarian cancer, testicular tumor, prostate cancer, thyroid cancer, kidney cancer, uterine cancer, etc.]. Furthermore, glycolipid derivative (I') or a salt thereof also enhances IFN-γ production through Mincle activation. Therefore, it is useful as an agent for the prophylaxis and/or treatment of bacterial infections, viral infections, and/or allergic diseases.

An embodiment of the glycolipid derivative (I') or a salt thereof of the present invention when used as the above-mentioned medicament is described in detail in the following.

"Prophylaxis" includes preventing the onset of a disease (all pathologies or symptoms, or one or more pathologies or symptoms) and delaying the onset of the disease. A "prophylactically effective amount" refers to a dose of glycolipid derivative (I') or a salt thereof sufficient to achieve this purpose. "Treatment" includes curing a disease (all pathologies or symptoms, or one or more pathologies or symptoms), improving the disease, and suppressing the progression of the disease severity. The "therapeutically effective amount" refers to a dose of glycolipid derivative (I') or a salt thereof sufficient to achieve this purpose.

Glycolipid derivative (I') or a salt thereof can be used alone or in the form of a pharmaceutical composition containing glycolipid derivative (I') or a salt thereof as the active ingredient together with a pharmaceutically acceptable carrier (hereinafter also referred to as "the present medicament").

Examples of the pharmaceutical composition include tablet (including sugar-coated tablet, film-coated tablet, sublingual tablet, orally disintegrating tablet, buccal tablet, etc.), pill, powder, granule, capsule (including soft capsule and microcapsule), troch, syrup, liquid, emulsion, suspension, controlled-release preparation (e.g., immediate-release preparation, sustained-release preparation, sustained-release microcapsule), aerosol, film (e.g., orally disintegrating film, oral mucosal patch film), injection (e.g., subcutaneous injection, intravenous injection (e.g., bolus), intramuscular injection, intraperitoneal injection), drip infusion, transdermal preparation, ointment, lotion, patch, suppository (e.g., rectal suppository, vaginal suppository), pellet, nasal preparation, pulmonary preparation (inhalant), eye drop, and the like.

The above-mentioned "pharmaceutically acceptable carrier" can be any of the various carriers conventionally used in the field of formulation technology.

Specific examples of the "pharmaceutically acceptable carrier" that can be used in solid preparations include excipients (e.g., lactose, sucrose, D-mannitol, starch, corn starch, crystalline cellulose, light anhydrous silicic acid, etc.), lubricants (e.g., magnesium stearate, talc, colloidal silica, etc.), binders (e.g., crystalline cellulose, sucrose, D-mannitol, dextrin, hydroxypropyl cellulose, hydroxypropylmethylcellulose, polyvinylpyrrolidone, starch, sucrose, gelatin, methylcellulose, sodium carboxymethylcellulose, etc.), disintegrants (e.g., starch, carboxymethylcellulose, calcium carboxymethylcellulose, sodium carboxymethylstarch, L-hydroxypropylcellulose, etc.), and the like.

In liquid preparations, solvents (e.g., water for injection, isotonic saline, alcohol, propylene glycol, macrogol, sesame oil, etc.), solubilizing agents (e.g., polyethylene glycol, propylene glycol, D-mannitol, benzyl benzoate, ethanol, triethanolamine, sodium carbonate, sodium citrate, etc.), suspending agents (e.g., surfactants such as stearyltriethanolamine, sodium lauryl sulfate, laurylaminopropionic acid, lecithin, benzalkonium chloride, glycerin monostearate, etc.; hydrophilic polymers such as polyvinyl alcohol, polyvinylpyrrolidone, sodium carboxymethylcellulose, methylcellulose, hydroxymethylcellulose, hydroxypropylcellulose, etc.), isotonic agents (e.g., glucose, D-sorbitol, sodium chloride, glycerin, D-mannitol, etc.), buffers (e.g., buffer solutions such as phosphate and citrate), soothing agents (e.g., benzyl alcohol, etc.), and the like can be used.

Where necessary, formulation additives such as preservatives (e.g., parahydroxybenzoic acid ester, chlorobutanol, benzyl alcohol, sorbic acid, etc.), antioxidants (e.g., sulfite, ascorbic acid, α-tocopherol, etc.), colorants, sweeteners, and the like may be further added.

While subject to change depending on the dosage form, administration method, carrier, and the like, the above-mentioned pharmaceutical composition can be produced by adding glycolipid derivative (I') or a salt thereof in a proportion of generally 0.01 to 99% (w/w), preferably 0.1 to 85% (w/w), based on the total amount of the preparation. The pharmaceutical composition can be produced by conventional methods in the field of formulation technology, according to the form thereof. The pharmaceutical compositions of the present invention may also be formed into a controlled-release preparation, such as immediate-release preparation or sustained-release preparation, containing the active ingredient.

As demonstrated by the representative compounds in the Experimental Examples described later, glycolipid derivative (I') and a salt thereof are expected to have low toxicity and few side effects, and also possess excellent properties as a pharmaceutical product. Therefore, the present medicament can be safely administered to any animal without limitation as long as it has an immune system, for example, mammals (e.g., human, monkey, dog, cow, horse, etc.), particularly human.

In practicing the present invention, glycolipid derivative (I') or a salt thereof, alone or as a pharmaceutical composition, can be administered orally or parenterally (e.g., intravenously, by infusion, intramuscularly, subcutaneously, intraviscerally, intranasally, intradermally, transdermally, by instillation, intracerebrally, rectally, intravaginally, intraperitoneally, and into a lesion).

The dosage of glycolipid derivative (I') or a salt thereof varies depending on the subject of administration, route of administration, and the age and symptom of the administration subject, and is not particularly limited. For example, the dosage of the active ingredient, glycolipid derivative (I') or a salt thereof, when administered orally, is 0.1 to 1000 mg, preferably 0.1 to 500 mg, more preferably 1 to 300 mg, per dose. The dosage can be divided into one to three doses per day. Furthermore, when it is administered in the form of a sustained-release preparation, it can be administered every other day or at longer intervals, corresponding to the dosage.

### (2) Use as adjuvant

As demonstrated by the representative compounds in the Experimental Examples described later, glycolipid derivative (I') or a salt thereof affords an immunostimulating action and can therefore be used as an adjuvant. In the present invention, "adjuvant" is a general term for a substance that can increase antibody production and enhance immune responses when combined with an antigen.

An embodiment of the glycolipid derivative (I') or a salt thereof of the present invention when used as an adjuvant is described in detail below.

When glycolipid derivative (I') or a salt thereof is used as an adjuvant, the dosage form thereof may be, for example, an aqueous or non-aqueous (e.g., oily, etc.) solution, suspension, emulsion, or the like. These can be prepared by mixing glycolipid derivative (I') or a salt thereof with a pharmaceutically acceptable carrier (e.g., solvent, suspending agent, etc.) and using methods such as manual shaking, mechanical shaking, ultrasonic dispersion, dispersion using a homomixer, self-emulsification, membrane emulsification, D-phase emulsification, vacuum emulsification, and ultra-high pressure emulsification (hereinafter also referred to as "the present adjuvant").

The present adjuvant may also be used in combination with other adjuvants. Examples of other adjuvants include adjuvants used in the art, such as Freund's incomplete adjuvant, Freund's complete adjuvant, microparticles (e.g., uric acid crystal, silica, aluminum hydroxide gel, polystyrene, asbestos, titanium dioxide, black nickel oxide, etc.), lipopolysaccharides (LPS), and the like.

The present invention also provides a vaccine containing the glycolipid derivative (I') of the present invention or a salt thereof and an antigen (hereinafter also referred to as "the present vaccine").

The antigen is not particularly limited as long as it is a substance capable of inducing an immune response, and examples thereof include (1) allergens (e.g., pollen allergen, food allergen, house dust allergen, etc.), (2) pathogen antigens (e.g., (i) pathogenic viral antigens (e.g., viral antigens such as human immunodeficiency virus, hepatitis virus, influenza virus, etc.), (ii) pathogenic microbial antigens (e.g., antigens expressed in pathogenic bacteria (e.g., Streptococcus pneumoniae, Clostridium tetani, Corynebacterium diphtheriae, Bordetella pertussis, Vibrio cholerae, Salmonella, S. Typhi, Chlamydia, Mycobacteria, Legionella, etc.), pathogenic yeasts (e.g., Aspergillus, Candida, etc.), etc.), (iii) pathogenic protozoan antigens (e.g., antigens expressed in malaria, Schistosoma, etc.), (3) in vivo self-antigens (e.g., amyloid beta and prions in neurological diseases such as Alzheimer's disease and Creutzfeldt-Jakob disease; ApoB100 in cardiovascular diseases such as arteriosclerosis and hypertension), (4) tumor antigens (e.g., antigens of solid tumors including epithelial and non-epithelial tumors, antigens of tumors in hematopoietic tissues), and the like.

The present vaccine can be administered by a route selected from the group consisting of oral administration, intramuscular administration, transdermal administration, intradermal administration, subcutaneous administration, intraperitoneal administration, intratracheal administration, nasal administration (intranasal administration), intraocular administration, intravaginal administration, rectal administration, intravenous administration, small intestinal administration, and inhalation administration, and subcutaneous administration and nasal administration (intranasal administration) are particularly preferred.

The antigen content in the present vaccine need only be an effective amount that functions as a vaccine, and such amount can be determined by one of ordinary skill in the art without undue experimentation, for example, based on tests using laboratory animals. Specifically, the content of antigen in the present vaccine is generally 1 to 100 µg.

The content of the glycolipid derivative (I') of the present invention or a salt thereof in the present vaccine can be adjusted appropriately according to, for example, the type of antigen, subject of administration, administration form, and administration route, and is not particularly limited. In the case of oral administration, intramuscular administration, transdermal administration, intradermal administration, subcutaneous administration, or intraperitoneal administration, the content is, for example, 2 µg to 1000 mg, generally 2 µg to 500 mg, preferably 2 µg to 20 mg, more preferably 20 µg to 200 µg. In the case of intratracheal administration, nasal administration (intranasal administration), intraocular administration, intravaginal administration, rectal administration, intravenous administration, small intestinal administration, or inhalation administration, the content is generally 0.01 µg to 1 mg, preferably 0.1 µg to 100 µg.

In addition to the glycolipid derivative (I') or a salt thereof and the antigen, the present vaccine may contain a pharmaceutically acceptable carrier. Examples of the pharmaceutically acceptable carriers that the present vaccine may contain include those exemplified as pharmaceutically acceptable carriers that the "present medicament" may contain.

The present vaccine may also contain other adjuvant. Examples of such other adjuvant include those exemplified as adjuvants that can be used in combination with the present adjuvant.

Examples of the dosage form for the present vaccine include those exemplified as the dosage form of the present medicament.

The present vaccine can be produced by methods conventionally used in the formulation technology field, for example, the method described in the 16th Edition of the Japanese Pharmacopoeia. For example, the present vaccine can be prepared by mixing the present medicament with the desired antigen and emulsifying or dispersing the mixture as necessary, or by adding the present medicament to a vaccine containing the desired antigen and emulsifying or dispersing the mixture as necessary.

The subject of administration of the present vaccine is not particularly limited as long as it is an animal having an immune system. Examples thereof include the same animals as those exemplified as the subject of administration of the present medicament.

The present vaccine may be administered as a single dose or multiple consecutive doses. When the present vaccine is administered consecutively, the administration period is not particularly limited and can be set appropriately according to, for example, the type of antigen, subject of administration, administration form, and administration route. It is generally in the range of 1 to 150 days, and can also be administered for 1 to 120 days, 1 to 60 days, or 1 to 30 days, where necessary.

By administering the present vaccine to a subject, allergic diseases, infectious diseases, cancer, and the like can be prevented and/or treated.

### [Example]

The present invention is described in more detail below according to Examples, but these examples are not intended to limit the scope of the present invention in any way. Furthermore, unless otherwise specified, the reagents, equipment, and materials used in the present invention are commercially available or can be prepared appropriately by those skilled in the art.

### [Production of glycolipid derivative (I) or a salt thereof]

### (1) General Experimental Procedure

### General Experimental Procedure A: Activation before glycosylation

The donor compound (1.5 eq), Ph₂SO (2.0 eq), and TTBP (3.8 eq) were dried by coevaporation with toluene and reduced pressure/nitrogen purge. The mixture was then dissolved in DCM (0.05 M), and flame-dried 3Å molecular sieves were added. The solution was then cooled to -60°C, and Tf₂O (2.0 eq) was added. After stirring for 30 min, the acceptor compound (1.0 eq), dried by coevaporation with toluene and reduced pressure/nitrogen purge, was dissolved in DCM (0.4 M) and slowly added to the solution. After TLC analysis confirmed the consumption of the acceptor compound (1-4 hr), the reaction was quenched by the addition of NEt₃. The reaction mixture was diluted with DCM, filtered through Celite, washed with brine, dried over MgSO₄, and concentrated under reduced pressure. The resultant product was then purified by column chromatography.

### General Experimental Procedure B: Sonogashira cross coupling

Iodo arylglycoside (1.0eq) was dissolved in freshly distilled NEt₃ (0.05 M) along with alkyne (1.2-3 eq). A mixture of Pd(PPh₃)₂Cl₂, PPh₃, and CuI (mixing ratio=1:1:2) was dissolved in freshly distilled NEt₃ and stirred at 40°C for 15 min. Of this mixture, amounts equivalent to 0.05 eq of Pd(PPh₃)₂Cl₂, 0.05 eq of PPh₃, and 0.1 eq of CuI were added to the sugar/alkyne mixture. The reaction product was stirred at 40°C until complete consumption of the starting material was confirmed by TLC (2-16 hr). The solvent was then evaporated under a stream of nitrogen. The crude product was purified by column chromatography on a silica column.

### General Experimental Procedure C: Esterification with mycocerosic acid

The starting material (1.0 eq) was dissolved in dry DCM (0.05 M) along with mycocerosic acid (3.0 eq) and DMAP (9 eq). The obtained mixture was cooled to 0°C, and DIC (6 eq) was added. The reaction product was stirred for 16 hr while warming to room temperature, then warmed to 40°C and stirred for an additional 5 hr. The reaction mixture was then diluted with Et₂O, and the organic layer was washed with 1M HCl, sat.aq.NaHCO₃, and brine, dried over MgSO₄, and concentrated under reduced pressure. The product was then purified by column chromatography.
Note: Staining with KMnO₄ was required to detect the most significant byproduct on TLC.

### General Experimental Procedure D: Hydrogenation

The starting material (1.0 eq) was dissolved in a mixture of THF and EtOH (1:1, 0.007 M), and the solution was purged with nitrogen. Next, Pd/C (10%, 1.0 eq) was added to the solution, and the obtained mixture was purged with H₂. The reaction product was stirred under a H₂ atmosphere until complete conversion of the starting material and reaction intermediate was confirmed on TLC (DCM:MeOH=19:1). The reaction mixture was then purged with nitrogen, filtered through Celite, and the Celite was rinsed with acetone. The resultant product was then purified by column chromatography.

### (2) Production of raw material compound

The production scheme of the raw material compounds are shown in the following formula. 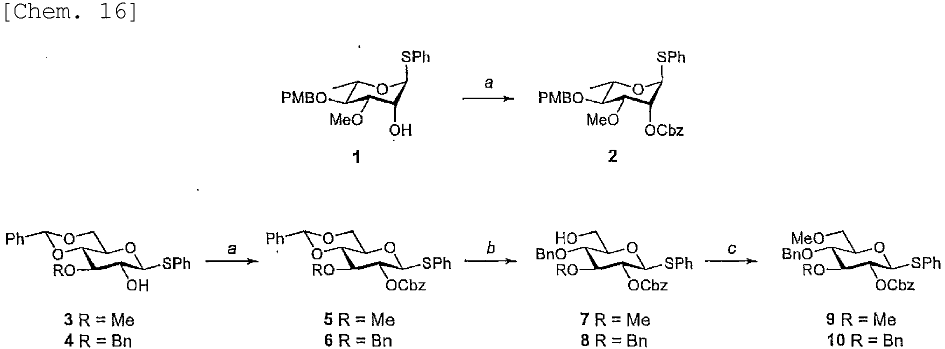 wherein, in the above-mentioned formula, Ph is a phenyl group, Me is a methyl group, PMB is a 4-methoxybenzyl group, Bn is a benzyl group, and Cbz is a benzyloxycarbonyl group.

### Production Example 1: Production of compound 2

Compound 1^{[1]} (144 mg, 0.37 mmol, 1.0eq) was dissolved in DCM (2 mL, 0.2 M), and DMAP (124 mg, 0.94 mmol, 2.5eq) was added to the solution. The mixture was cooled to 0°C, and CbzCl (0.11 mL, 0.75 mmol, 2.0eq) was slowly added. The reaction product was stirred for 4 hr while slowly warming to room temperature. The reaction was then quenched by the addition of 1 MHCl, the organic layer was washed with sat.aq.NaHCO₃ and brine, dried over MgSO₄, filtered, and concentrated under reduced pressure. It was purified by column chromatography (n-pentane:Et₂O=4:1) to give compound 2 (184 mg, 0.35 mmol, 94%) as a transparent oily substance.
HRMS calculated for C₂₉H₃₆NO₇S 542.2212 [M+NH₄]⁺; found 542.2208

### Production Example 2: Production of compound 5

Compound 3^{[1]} (0.57 g, 1.41 mmol,1.0eq) was dissolved in DCM (14 mL, 0.1 M), and DMAP (0.38 g, 3.10 mmol, 2.2eq) was added to the solution. The mixture was cooled to 0°C, and CbzCl (0.4 mL, 2.82 mmol, 2.0eq) was slowly added. The reaction product was stirred for 6 hr while slowly warming to room temperature. The reaction was then quenched by the addition of 1 M HCl, and the organic layer was washed with sat.aq.NaHCO₃ and brine, dried over MgSO₄, and concentrated under reduced pressure. It was purified by column chromatography (n-pentane:Et₂O=4:1) to give compound 5 (0.637 g, 1.25 mmol, 89%) as a white solid.
HRMS calculated for C₂₈H₂₈O₇SNa 531.1453 [M+Na]⁺; found 531.1444

### Production Example 3: Production of compound 7

Compound 5 (0.63 g, 1.24 mmol, 1.0eq.) was coevaporated with toluene under a N₂ atmosphere and then dissolved in dry DCM (12.4 mL, 0.1 M). BH₃·THF (1 M in THF, 6.2 mL, 6.2 mmol, 5.0eq) was added dropwise to the solution, and then TMSOTf (22 µL, 0.12 mmol, 0.1eq) was added to the mixture. The reaction mixture was stirred for 5 hr, and slowly quenched by adding NEt₃ (1.2 mL), followed by MeOH, until H₂ formation ceased. The mixture was concentrated, and coevaporated with MeOH. It was purified by column chromatography (n-pentane:Et₂O=7:3) to give compound 7 (0.628 g, 1.23 mmol, 99%) as a white solid.
HRMS calculated for C₃₅H₃₆O₇SNa 533.1610 [M+Na]⁺; found 533.1605

### Production Example 4: Production of compound 9

Compound 7 (179 mg, 0.35 mmol, 1.0eq) and TTBP (362 mg, 1.46 mmol, 4.0eq) were coevaporated with toluene under a N₂ atmosphere, dissolved in dry DCM (7.2 mL, 0.05 M), and flame-dried 3Å molecular sieves were added. Trimethyloxonium tetrafluoroborate (160 mg, 1.08 mmol, 3.0 eq) was then added to the mixture, and the reaction product was stirred for 1.5 hr. The reaction was quenched with NEt₃ (0.5 mL), and the mixture was filtered through Celite, washed with brine, dried over MgSO₄, and concentrated under reduced pressure. It was purified by column chromatography (n-pentane:Et₂O=4:1) to give compound 9 (143 mg, 0.27 mmol, 78%) as a white solid.
HRMS calculated for C₂₉H₃₂O₇SNa 547.1766 [M+Na]⁺; found 547.1761

### Production Example 5: Production of compound 6

Compound 4^{[1]} (2.03 g, 4.51 mmol, 1.0eq) was dissolved in DCM (173 mL, 0.03 M), and DMAP (1.65 g, 13.5 mmol, 3.0eq) was added to the solution. The mixture was cooled to 0°C, and CbzCl (1.9 mL, 13.5 mmol, 3.0eq) was slowly added. The reaction product was stirred for 5 hr while slowly warming to room temperature. The reaction was then quenched by the addition of 1 MHCl, and the organic layer was washed with sat.aq.NaHCO₃ and brine, dried over MgSO₄, and concentrated under reduced pressure. It was purified by column chromatography (n-pentane:Et₂O=4:1) to give compound 6 (2.14 g, 3.66 mmol, 81%) as a white solid.
HRMS calculated for C₃₄H₃₂O₇SNa 607.1766 [M+Na]⁺; found 607.1761

### Production Example 6: Production of compound 8

Compound 6 (257 mg, 0.44 mmol, 1.0eq.) was coevaporated with toluene under a N₂ atmosphere and then dissolved in dry DCM (4.4 mL, 0.1 M). A 1 M solution of BH₃·THF (4.4 mL, 4.4 mmol, 10.0 eq) in THF was added dropwise to the solution, and TMSOTf (0.08 mL, 0.44 mmol, 1.0 eq) was added to the mixture. The reaction mixture was stirred for 4 hr and slowly quenched by adding NEt₃ (1 mL), and then MeOH, until H₂ formation ceased.
The mixture was concentrated, and coevaporated with MeOH. It was purified by column chromatography (n-pentane:Et₂O=7:3) to give compound 8 (0.234 g, 0.40 mmol, 91%) as a white solid.
HRMS calculated for C₃₄H₃₄O₇SNa 609.1923 [M+Na]⁺; found 609.1918

### Production Example 7: Production of compound 10

Compound 8 (135 mg, 0.23 mmol, 1.0eq) and TTBP (229 mg, 0.92 mmol, 4.0eq) were coevaporated with toluene under a N₂ atmosphere, dissolved in dry DCM (4.6 mL, 0.05 M), and flame-dried 3Å molecular sieves were added. Next, trimethyloxonium tetrafluoroborate (102 mg, 0.69 mmol, 3.0 eq) was added to the mixture, and the reaction product was stirred for 2 hr. The reaction was quenched with NEt₃ (0.5 mL), filtered through Celite, washed with brine, dried over MgSO₄, and concentrated under reduced pressure. It was purified by column chromatography (n-pentane:Et₂O=4:1) to give compound 10 (99 mg, 0.165 mmol, 72%) as a white solid.
HRMS calculated for C₃₅H₃₆O₇SNa 623.2079 [M+Na]⁺; found 623.2074

### (3) Production of oligo saccharide

The production scheme of oligosaccharide is shown in the following formula. 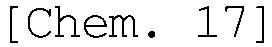 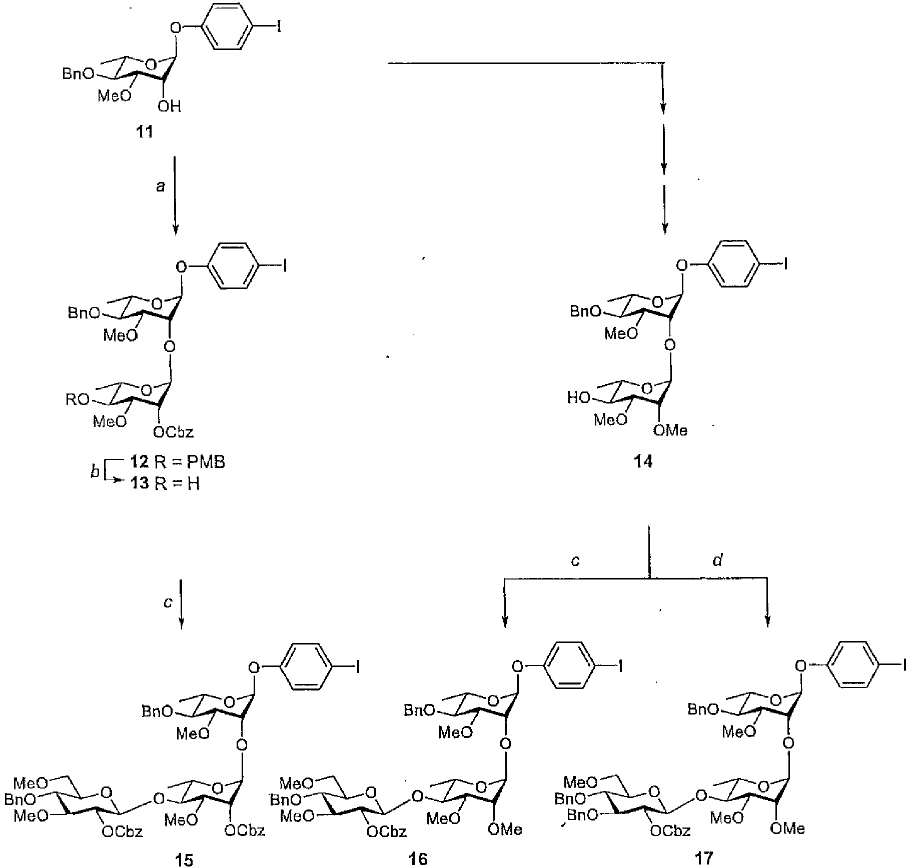 wherein, in the above-mentioned formula, Me, PMB and Cbz are each as defined above, and Bn is a benzyl group.

### Production Example A: Production of compound 12

Using compound 2 (393 mg, 0.75 mmol, 1.5eq) and compound 11^{[1]} (235 mg, 0.5 mmol, 1.0eq) and according to glycosylation procedure A, the object compound was prepared. The compound was purified by column chromatography (n-pentane:Et₂O=4:1) to give compound 12 as a slightly-yellow oily substance (206 mg, 0.23 mmol, 47%). HRMS calculated for C₄₃H₄₉IO₁₂Na 907.2166 [M+Na]⁺; found 907.2143

### Production Example B: Production of compound 13

Compound 12 (192 mg, 0.22 mmol, 1.0eq) was dissolved in a mixture of DCM and HFIP (1:1, 2.2 mL, 0.1 M), and then a solution of hydrochloric acid (0.11 mL, 0.2 M, 0.1 eq) in HFIP was added. After complete conversion of the starting material, indicated by a deep purple color, the reaction was quenched by the addition of sat.aq.NaHCO₃. The mixture was diluted with DCM, washed with brine, dried over MgSO₄, and concentrated under reduced pressure. It was purified by column chromatography (n-pentane:Et₂O=1:1) to give compound 13 (163 mg, 0.21 mmol, 98%) as a pale oily substance.
HRMS calculated for C₃₅H₄₁IO₁₁Na 787.1591 [M+Na]⁺; found 787.1567

### Production Example C: Production of compound 15

Using compound 9 (67 mg, 0.12 mmol, 1.5eq) and compound 13 (63 mg, 0.08 mmol, 1.0eq) and according to glycosylation procedure A, the object compound was prepared. The compound was purified by column chromatography (n-pentane:Et₂O=3:2) to give compound 15 as a slightly-yellow oily substance (66 mg, 0.06 mmol, 68%).
HRMS calculated for C₅₈H₆₇IO₁₈Na 1201.3270 [M+Na]⁺; found 1201.3257

### Production Example D: Production of compound 16

Using compound 9 (2.57 g, 4.9 mmol, 1.4eq) and compound 14^{[1]} (2.23 g, 3.47 mmol, 1.0eq) and according to glycosylation procedure A, the object compound was prepared. The compound was purified by column chromatography (n-pentane:Et₂O=2:3) to give compound 16 as a light oily substance (2.93 g, 2.77 mmol, 80%). HRMS calculated for C₅₁H₆₃IO₁₆Na 1081.3058 [M+Na]⁺; found 1081.3053

### Production Example E: Production of compound 17

Using compound 10 (105 mg, 0.17 mmol, 1.5eq) and compound 14^{[1]} (91 mg, 0.12 mmol) and according to glycosylation procedure A, the object compound was prepared. The compound was purified by column chromatography (n-pentane:Et₂O=1:4) to give compound 17 as a slightly-yellow oily substance (127 mg, 0.11 mmol, 96%). HRMS calculated for C₅₇H₆₇IO₁₆Na 1157.3371 [M+Na]⁺; found 1157.3366

### (4) Production of glycolipid derivative

### 1) Production of PGL-I compound to III compound (PGLs)

The production scheme is shown in the following formula. 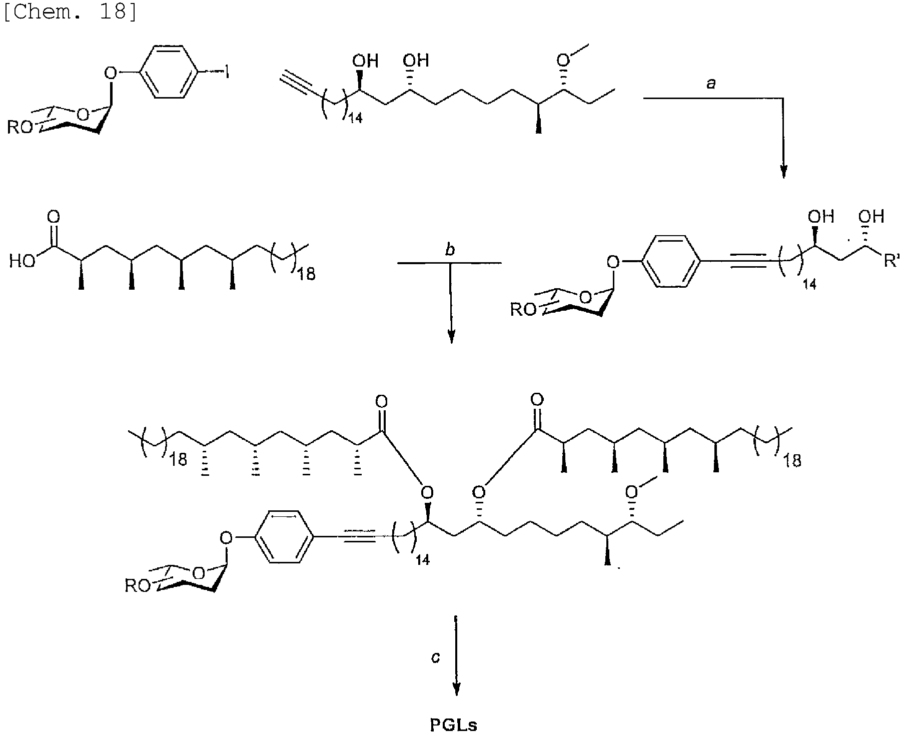 wherein, in the above-mentioned formula, compounds represented by 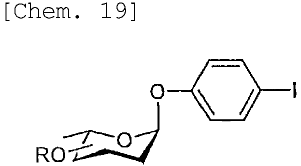 are compounds 15 to 17 produced in Production Examples C to E, respectively.

The structural formulas of the produced PGL-I compound (Example 1), PGL-II compound (Example 2), and PGL-III compound (Example 3) are shown in the following.

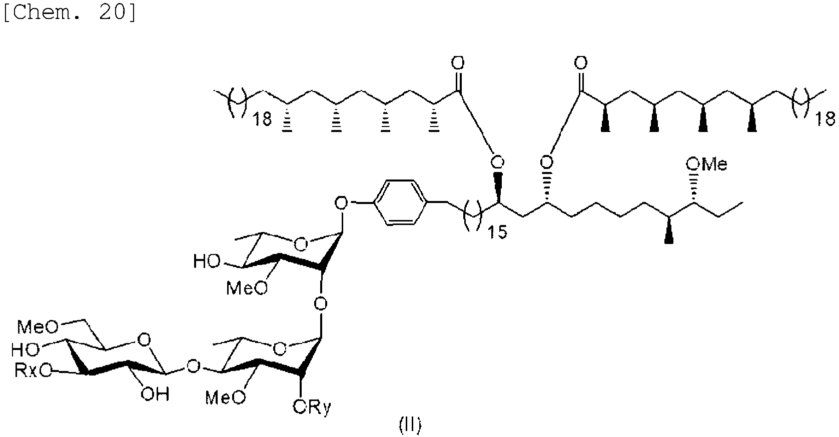

**[Table 3]**

| glycolipid derivative | Rx | Ry |
|---|---|---|
| PGL-I compound | methyl group | methyl group |
| PGL-II compound | methyl group | hydrogen atom |
| PGL-III compound | hydrogen atom | methyl group |

### Example 1: Production of PGL-I

### (a) compound 18

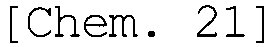

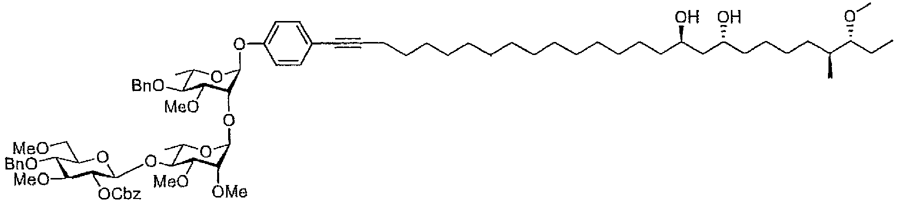

Using compound 16 (23 mg, 22 µmol, 1.0eq) and phthiocerol^{[2]} (12 mg, 26 µmol, 1.2eq) and according to general experimental procedure B, compound 18 was synthesized. The compound was purified by column chromatography (DCM:acetone=4:1) to give a resultant product (25 mg, 18 µmol, 83%) as a yellow oily substance.
HRMS calculated for C₈₀H₁₁₈O₁₉Na 1405.8165 [M+Na]⁺; found 1405.8160

### (b) compound 21

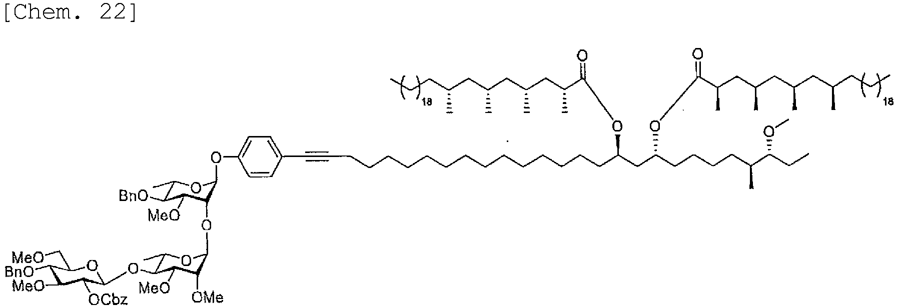

Using compound 18 (34 mg, 25 µmol, 1.0eq), mycocerosic acid ^{[3]} (35 mg, 74 µmol, 3.0eq), DIC (23 µL, 147 µmol, 6.0eq) and DMAP (27 mg, 221 µmol, 9.0eq) and according to general experimental procedure C, compound 21 was synthesized. The compound was purified by column chromatography (n-pentane:Et₂O=1:1) to give a resultant product (45 mg, 19 µmol, 79%) as a waxy solid.
HRMS calculated for C₁₄₄H₂₄₃O₂₁2309.79755 [M+H]⁺; found 2309.80566

### (c) compound 24 (PGL-I compound; structural formula is as shown above)

Using compound 21 (32 mg, 14 µmol, 1.0eq) and Pd/C (10%, 15 mg, 14 µmol, 1.0eq) and according to general experimental procedure D, compound 24 was synthesized. The compound was purified by column chromatography (DCM:acetone=3:2) to give PGL-I (22 mg, 11 µmol, 79%) as a waxy solid.
[α]D²⁵ = -25.2 (c = 1.0, CHCl₃)
¹H-NMR (850 MHz) δ:7.10 (d, 2H, J = 9.4 Hz, CHₐᵣₒₘ); 6.94 (d, 2H, J = 8.5 Hz, CHₐᵣₒₘ); 5.43 (d, 1H, J = 1.7 Hz, H-1); 5.10 (d, 1H, J = 1.7 Hz, H-1'); 4.84 (quint, 2H, J = 6.4 Hz, CH_{Phth}); 4.41 (d, 1H, J = **7.7** Hz, H-1"); 4.22 (dd, 1H, J= 1.7, 3.4 Hz, H-2); 3.89 (s, 1H, 2"-OH); 3.77-3.74 (m, 3H, H-2', H-5, H-5'); 3.69-3.66 (m, 4H, H-3',OCH₃); 3.65-3.61 (m, 4H, H-3, H-4', H-6"); 3.58 (dt, 1H, J = 1.7, 9.4 Hz, H-4); 3.55-3.52 (m, 4H, H-4",OCH₃); 3.50 (s, 3H, OCH₃); 3.48 (s, 3H, OCH₃); 3.38 (s, 3H, OCH₃); 3.17 (t, 1H, J = 9.4 Hz, H-3"); 2.87-2.84 (m, 1H, CH_{Phth}); 2.80 (bs, 1H, 4"-OH); 2.56-2.51(m, 4H, CH2,_{Phth}, CH_{Myc}); 2.30 (bs, 1H, 4-OH); 1.77-0.81 (m, 190H, H-6, H-6', CH_{Phth}, CH2, _{Phth}, CH₃, _{Phth}, CH_{Myc}, CH_{2,Myc}, CH_{3,Myc}) HRMS calculated for C₁₂₂H₂₂₉O₁₉1998.69476 [M+H]⁺; found 1998.69683

### Example 2: Production of PGL-II

### (a) compound 19

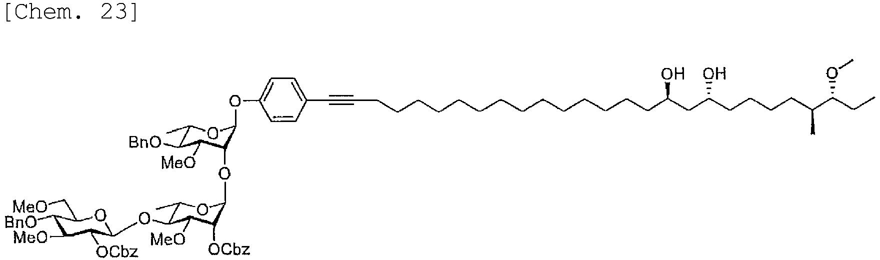

Using compound 15 (59 mg, 50 µmol, 1.0eq) and phthiocerol^{[2]} (27 mg, 60 µmol, 1.2eq) and according to general experimental procedure B, compound 19 was synthesized. The compound was purified by column chromatography (DCM:acetone=4:1) to give a resultant product (70 mg, 47 µmol, 93%) as a yellow oily substance.
HRMS calculated for C₈₇H₁₂₂O₂₁Na 1525.8376[M+Na]⁺; found 1525.8374

### (b) compound 22

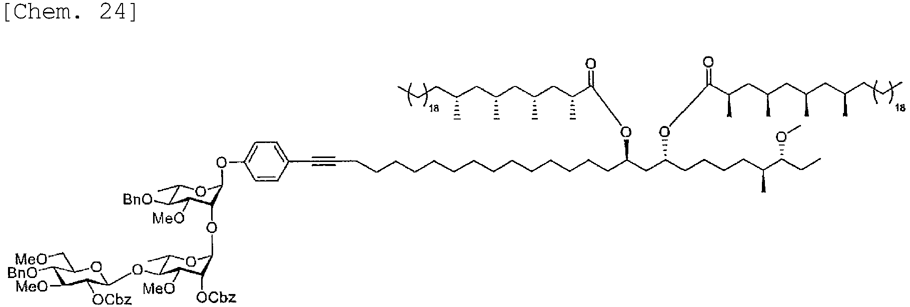

Using compound 19 (37 mg, 25 µmol, 1.0eq), mycocerosic acid ^{[3]} (35 mg, 74 µmol, 3.0eq), DIC (23 µL, 148 µmol, 6.0eq) and DMAP (27 mg, 221 µmol, 9.0eq) and according to general experimental procedure C, compound 22 was synthesized. The compound was purified by column chromatography (n-pentane:Et₂O=4:1) to give a resultant product (48 mg, 20 µmol, 80%) as a waxy solid.
HRMS calculated for C₁₅₁H₂₄₇O₂₃2429.81868 [M+H]⁺; found 2429.82801

### (c) compound 25 (PGL-II compound; structural formula is as shown above)

Using compound 22 (37 mg, 15 µmol, 1.0eq) and Pd/C (10%, 16 mg, 15 µmol, 1.0eq) and according to general experimental procedure D, compound 25 was synthesized. The compound was purified by column chromatography (DCM:MeO=11:1) to give PGL-II (23 mg, 12 µmol, 76%) as a waxy solid.
[α]D²⁵ = -24.4 (c = 1.0, CHCl₃)
¹H-NMR (850 MHz) δ:7.10 (d, 2H, J = 8.5 Hz, CHₐᵣₒₘ); 6.95-6.94 (m, 2H, CHₐᵣₒₘ); 5.46 (d, 1H, J = 1.7 Hz, H-1) ; 5.08 (d, 1H, J= 1.7 Hz, H-1'); 4.84 (quint, 2H, J = 6.4 Hz, CH_{Phth}) ; 4.39 (d, 1H, J = 7.7 Hz, H-1"); 4.22 (dd, 1H, J = 2.4, 2.8 Hz, H-2); 4.20 (s, 1H, H-2'); 3.82 (dq, 1H, J = 3.4, 6.0 Hz, H-5'); 3.76 (dq, 1H, J = 3.4, 6.0 Hz, H-5); 3.71 (s, 1H, 2"-OH); 3.69 (s, 3H, OCH₃); 3.66-3.63 (m, 4H, H-3, H-3', H-6''); 3.61-3.57 (m, 2H, H-4, H-4'); 3.55 (dt, 1H, J = 2.0, 8.9 Hz, H-4"); 3.51 (s, 3H, OCH₃); 3.51 (s, 3H, OCH₃); 3.45-3.39 (m, 2H, H-2",H-5") ; 3.33 (s, 3H, OCH₃) ; 3.18 (t, 1H, J = 8.9 Hz, H-3") ; 2.87-2.85 (m, 2H, CH_{Phth}, 4"-OH); 2.55-2.53 (m, 4H, CH₂,_{Phth}, CH_{Myc}); 2.34 (bs, 2H, 4-OH, 2'-OH); 1.77-0.81 (m, 189H, H-6, H-6', CH_{Phth}, CH₂,_{Phth}, CH_{3,Phth}, CH_{Myc}, CH₂, _{Myc}, CH_{3, Myc})
HRMS calculated for C₁₂₁H₂₂₇O₁₉1985.68253 [M+H]⁺; found 1985.68284

### Example 3: Production of PGL-III

### (a) compound 20

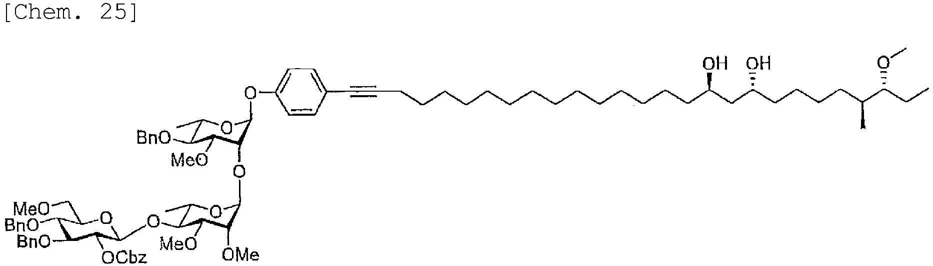

Using compound 17 (62 mg, 55 µmol, 1.0eq) and phthiocerol^{[2]} (30 mg, 66 µmol, 1.2eq) and according to general experimental procedure B, compound 20 was synthesized. The compound was purified by column chromatography (DCM:EtOAc=1:1) to give a resultant product (66 mg, 45 µmol, 83%) as a yellow oily substance.
HRMS calculated for C₈₆H₁₂₂O₁₉Na 1481.8478 [M+Na]⁺; found 1481.8473

### (b) compound 23

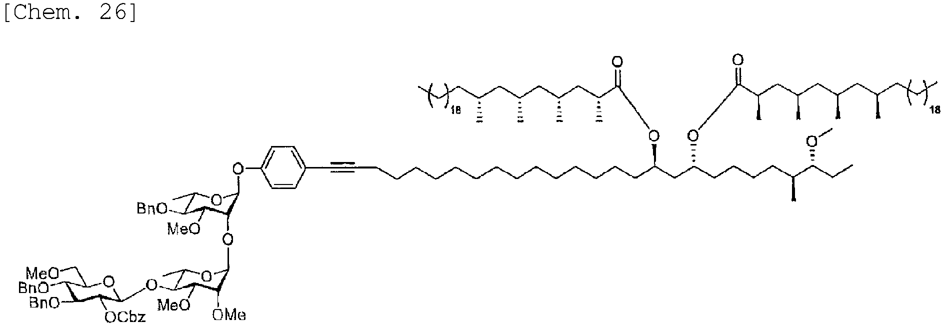

Using compound 20 (26 mg, 18 µmol, 1.0eq), mycocerosic acid ^{[3]} (25 mg, 53 µmol, 3.0eq), DIC (16 µL, 105 µmol, 6.0eq), and DMAP (19 mg, 158 µmol, 9.0eq) and according to general experimental procedure C, compound 23 was synthesized. The compound was purified by column chromatography (n-pentane:Et₂O=1:1) to give a resultant product (32 mg, 13.4 µmol, 76%) as a waxy solid.
HRMS calculated for C₁₅₀H₂₄₇O₂₁2385.82885 [M+H]⁺; found 2385.83921

### (c) compound 26 (PGL-III compound; structural formula is as shown above)

Using compound 23 (24 mg, 10 µmol, 1.0eq) and Pd/C (10%, 11 mg, 10 µmol, 1.0eq) and according to general experimental procedure D, compound 26 was synthesized. The compound was purified by column chromatography (DCM:MeOH=19:1) to give PGL-III (8 mg, 4 µmol, 40%) as a waxy solid.
[α]D²⁵ = -28 (c = 0.2, CHCl₃)
¹H-NMR (400 MHz) δ: 7.10(d, 2H, J = 8.8 Hz, CHₐᵣₒₘ); 6.94 (d, 2H, J = 8.8 Hz, CHₐᵣₒₘ); 5.43 (d, 1H, J = 2.0 Hz, H-1); 5.11 (d, 1H, J = 1.6 Hz, H-1'); 4.84 (quint, 2H, J = 6.4 Hz, CH_{Phth}); 4.45 (d, 1H, J = 7.6 Hz, H-1"); 4.23 (dd, 1H, J =1.6, 2.8 Hz, H-2); 3.99 (bs, 1H, OH); 3.79-3.71 (m, 3H, H-2', H-5, H-5'); 3.69-3.60 (m, 5H, H-3, H-3', H-4', H-6"); 3.58-3.54 (m, 6H, H-3", H-4, H-5", OCH₃); 3.50 (s, 3H, OCH₃); 3.49 (s, 3H, OCH₃); 3.46-3.42 (m, 1H, H-4"); 3.39-3.36 (m, 4H, H-2", OCH₃); 3.35 (s, 3H, OCH₃); 2.98 (bs, 1H, OH); 2.88-2.79 (m, 2H, CH_{Phth}, OH) ; 2.57-2.50 (m, 4H, CH2, _{Phth}, CH_{Myc}); 2.32 (bs, 1H, OH) ; 1.77-0.81 (m, 207H, H-6, H-6', CH_{Phth}, CH₂, _{Phth}, CH₃, _{Phth}*,* CH_{Myc}*,* CH_{2, Myc}, CH_{3, Myc}, )
HRMS calculated forC₁₂₁H₂₂₇O₁₉1985.68253 [M+H]⁺; found 1985.68265

### 2) Production of PGL analogue compound

### Example 4: Production of compound 27

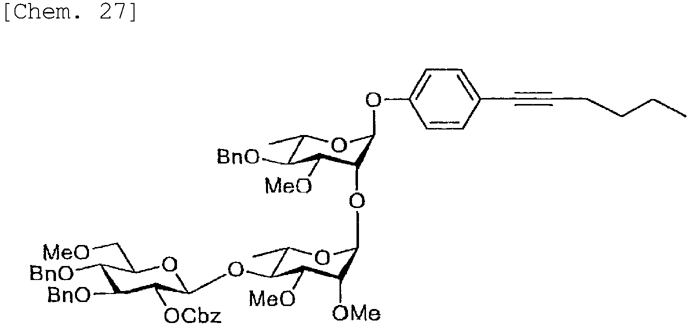

Using compound 17 (46 mg, 41 µmol, 1.0eq) and 1-hexyne (14 µL, 122 µmol, 3.0eq) and according to general experimental procedure B, compound 27 was synthesized. The compound was purified by column chromatography (n-pentane:Et₂O=4:6) to give a resultant product (40 mg, 37 µmol, 91%) as a yellow oily substance.
[α]D²⁵ = -60.9 (c = 1.0, CHCl₃)
¹H-NMR (400 MHz) δ: 7.37-7.21 (m, 22H, CHₐᵣₒₘ); 6.96-6.94 (m, 2H, CHₐᵣₒₘ) ; 5.48 (d, 1H, J = 1.6 Hz, H-1); 5.24-5.15 (m, 3H, H-1', PhCH_{2,Cbz}); 4.89 (d, 1H, J = 11.2 Hz, PhCHH); 4.80-4.76 (m, 4H, PhCHH, PhCHH, H-1", H-2"); 4.70-4.62 (m, 3H, PhCHH, PhCHH, PhCHH); 4.25 (dd, 1H, J = 2.4, 2.8 Hz, H-2); 3.79 (dd, 1H, J = 3.0, 9.4 Hz, H-3); 3.76-3.61 (m, 7H, H-2', H-3'', H-4'', H-5, H-5', H-5'', H-6"); 3.57-3.43 (m, 9H, H-4, H-4', H-6'', OCH₃); 3.39-3.32 (m, 7H, H-3', OCH₃); 2.39 (t, 1H, J = 7.0 Hz, CH₂); 1.63-1.52 (m, 2H, CH₂); 1.51-1.42 (m, 2H, CH₂); 1.32-1.25 (m, 6H, H-6, H-6'); 0.94 (t, 3H, J = 7.4 Hz, CH₃)
HRMS calculated for C₆₃H₇₆O₁₆Na 1111.50256 [M+Na]⁺; found 1111.50185

### Example 5: Production of compound 28 (HD-275)

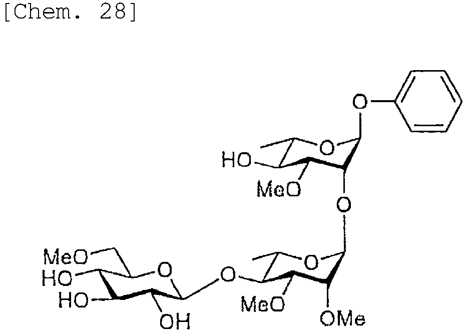

Compound 17 (34 mg, 30 µmol, 1.0eq) was hydrogenated using general experimental procedure D to give compound 28 (HD-275) (14 mg, 23 µmol, 77%) as a pale oily substance.
[α]D²⁵ = -53.7 (c =1.0, CHCl₃)
¹H-NMR (400 MHz) δ: 7.33-7.28 (m, 2H, CHₐᵣₒₘ); 7.05-7.02 (m, 3H, CHₐᵣₒₘ); 5.49 (d, 1H, J = 1.6 Hz, H-1); 5.11 (d, 1H, J = 1.2 Hz, H-1'); 4.45 (d, 1H, J = 7.6 Hz, H-1"); 4.24 (dd, 1H, J = 1.6, 3.2 Hz, H-2); 4.12 (bs, 1H, OH); 3.79-3.59 (m, 8H, H-2', H-3, H-3', H-4', H-5, H-5', H-6"); 3.57-3.35 (m, 18H, H-2", H-3", H-4, H-4", H-5", OCH₃); 2.52 (bs, 1H, OH); 1.34 (d, 3H, J = 6.4 Hz, H-6'); 1.27 (d, 3H, J = 6.0 Hz, H-6)
HRMS calculated for C₂₈H₄₄O₁₄Na627.26233 [M+Na]⁺; found 627.26222

### Example 6: Production of compound 29 (HD-276)

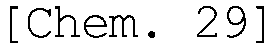

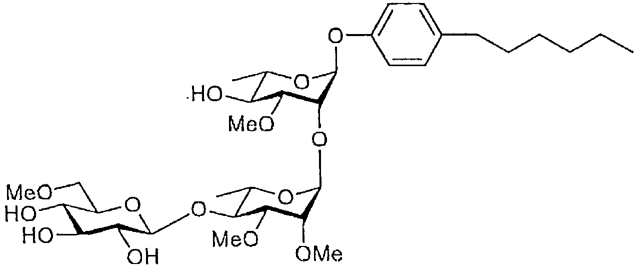

Compound 27 (32 mg, 29 µmol, 1.0eq) was hydrogenated using general experimental procedure D to give compound 29 (HD-276) (15 mg, 22 µmol, 74%) as a pale oily substance.
[α]D²⁵ = -50.3 (c = 1.0, CHCl₃)
¹H-NMR (400 MHz) δ:7.10 (d, 2H, J = 8.4 Hz, CHₐᵣₒₘ); 6.95 (dd, 2H, J = 2.0, 6.8 Hz, CHₐᵣₒₘ); 5.44 (d, 1H, J = 2.0 Hz, H-1); 5.10 (d, 1H, J = 1.6 Hz, H-1'); 4.45 (d, 1H, J = 7.6 Hz, H-1"); 4.24 (dd, 1H, J = 2.0, 2.8 Hz, H-2); 4.09 (bs, 1H, OH); 3.78-3.59 (m, 8H, H-2', H-3, H-3', H-4', H-5, H-5', H-6''); 3.57-3.35 (m, 18H, H-2", H-3", H-4, H-4", H-5", OCH₃); 3.21 (bs, 2H, OH); 2.55 (t, 2H, J = 7.6 Hz, CH₂); 2.45 (bs, 1H, OH); 1.58 (quint,2H, J = 7.2 Hz, CH₂); 1.33-1.25 (m, 12H, CH₂, H-6, H-6'); 0.88 (t, 3H, J = 6.8 Hz, CH₃)
HRMS calculated for C₃₄H₅₆O₁₄Na 711.35623 [M+Na]⁺; found 711.35585

The references cited in the above are as follows:
[1] Chem Bio Chem, vol. 22, no. 8, pp. 1487-1493, 2021
[2] Angewandte Chemie International Edition, vol. 124, no. 47, pp. 11774-11777, 2012
[3] Chem. Commun., no. 5, pp. 489-491, 2007

### [Pharmacological evaluation of glycolipid derivative (I') or a salt thereof]

In order to demonstrate the usefulness of glycolipid derivative (I') or a salt thereof, various pharmacological evaluation results are shown below.

### Test Compound

The test compounds subjected to the pharmacological evaluation are as follows:
PGL-I compound (glycolipid derivative of Example 1)
PGL-II compound (glycolipid derivative of Example 2)
PGL-III compound (glycolipid derivative of Example 3)
HD-275 (glycolipid derivative of Example 5)
HD-276 (glycolipid derivative of Example 6)
TDM (trehalose dimycolate); glycolipid derivative that is a known Mincle ligand
TDB (trehalose dibehenate): glycolipid derivative that is a known Mincle ligand

### Experimental Example 1: evaluation of Mincle ligand activity

NFAT-GFP reporter cells expressing Murine Mincle were prepared according to the method described in a literature (Yamasaki, S. et al, Nat Immunol 9, 1179-88(2008)). Test compounds (PGL-III compound and TDM; n=3 each) were immobilized on a 96-well plate, and cells were added. After 20 hr of stimulation, reporter activity was analyzed and the ligand activity of the test compounds was evaluated. The evaluation results are shown in Fig. 1 (left). The doses of the test compounds (horizontal axis) were 10⁻⁵, 10⁻⁴, 10⁻³, 10⁻², 10⁻¹, 10⁰, and 10¹ (nmol) from the left to right.

Similarly, using NFAT-GFP reporter cells expressing bovine Mincle, prepared according to the method described in a literature (Yamasaki, S. et al, Nat Immunol 9, 1179-88(2008)), the ligand activity of HD-276 was evaluated on a 96-well plate. The evaluation results are shown in Fig. 1 (right). The doses of the test compounds (horizontal axis) were 0.03 to 3 (nmol) from the left to right.

This evaluation clarified that the PGL-III compound has superior Mincle ligand activity to TDM. HD-276 was also confirmed to have Mincle ligand activity in a similar evaluation. Furthermore, in a similar evaluation, neither the PGL-I compound nor the PGL-II compound was found to have ligand activity.

From the forgoing facts, it can be understood that a hydroxy group at the 3-position of the terminal sugar represented by ring S1 of the glycolipid derivative (I') of the present invention is an essential structure for affording Mincle ligand activity.

### Experimental Example 2: evaluation of macrophage activation

Bone marrow-derived macrophages from wild-type mouse and Mincle-deficient mouse were prepared according to the method described in a literature (Kiyotake, R. et al, J Biol Chem 290, 25322-25332(2015); Ishikawa, T. et al, Cell Host Microbe 13, 477-488(2013)). Mincle-deficient mice were generated according to the method described in a literature (Yamasaki, S. et al, Proc Natl Acad Sci USA 106,1897-1902(2009)). Macrophages were stimulated for 48 hr with plate-immobilized test compounds (PGL-III and TDM; n=3 each), the amount of tumor necrosis factor (TNF) produced was analyzed, and the action of the test compound on immune responses was evaluated. The evaluation results are shown in Fig. 2. The doses of the test compounds are as shown in Fig. 2. LPS (0.1 µg/ml) was used as a positive control.

This evaluation clarified that PGL-III compound activates macrophages through Mincle activation and enhances TNF production in a dose-dependent manner, and further that the action thereof is superior to that of TDM.

### Experimental Example 3: analysis of crystal structure of Mincle-ligand complex

Using a complex of Mincle and a PGL-III analog (HD-276), the crystal structure analysis was performed. The analysis results are shown in Fig. 3 and Fig. 4. Fig. 3 shows an electron density map of the complex. Fig. 4 shows a schematic diagram of the binding mode between Mincle and PGL-III analog.

This structural analysis suggests that, in the glycolipid derivative (I') of the present invention, the glycan portion (oligosaccharide portion consisting of ring S1-ring S2-ring S3) and the lipid portion (R_{L} portion) each interact with Mincle, inducing efficient Mincle polymerization.

### Experimental Example 4: evaluation of immunostimulatory action

### (1) Examination of effect of enhancing antigen-specific IgG production

Using ovalbumin (OVA) as a model antigen, the adjuvant activity of the test compounds (PGL-III compound and TDB) was evaluated using the amount of IgG antibody production when administered to C57BL/6 mice as an indicator.

C57BL/6 mice were immunized with (i) OVA alone (OVA-only administration group; n=7), (ii) OVA and PGL-III compound (OVA+PGL-III compound administration group; n=5), or (iii) OVA and TDB (OVA+TDB administration group; n=7). For each group, mouse serum was collected over time after immunization, and the amount of OVA-specific IgG antibody produced was evaluated and quantified by ELISA, using a standard curve drawn using serum collected from OVA/Alum-immunized mice (*p<0.05, **p<0.01, ***p<0.005). The evaluation results are shown in Fig. 5.

This evaluation clarified that IgG antibody production is significantly enhanced in the OVA+PGL-III compound administration group compared to the OVA-only administration group, and that the degree of enhancement is greater than in the OVA+TDB administration group.

This fact clearly demonstrates that PGL-III compound has a superior immunostimulatory action and is superior as an adjuvant.

### (2) Examination of influence on body weight

In the above-mentioned test, changes in body weight were observed in mice in each administration group in parallel with the measurement of antibody amount. The evaluation results are shown in Fig. 6.

As with the other administration groups, no weight loss was observed in the OVA+PGL-III compound administration group, suggesting that the administration of PGL-III compound does not induce adverse events.

### Experimental Example 5: evaluation of cellular immunity-enhancing effect

Inguinal lymph node cells from immunized mice were pooled for each administration group (at least n=5 per group) and restimulated with OVA antigen for 72 hr. The amount of IFN-γ production was evaluated. The evaluation results are shown in Fig. 7.

This evaluation clarified that IFN-γ production is enhanced in the OVA+PGL-III compound administration group compared to the OVA alone administration group, and the degree of enhancement is greater than in the OVA+TDB administration group.

This fact clearly demonstrates that PGL-III compound is an adjuvant that can enhance not only humoral immunity but also cellular immunity.

### [Industrial Applicability]

The present invention relates to embodiments of glycolipid derivative and the like that are ligands for Mincle, which plays an important role in immune responses, and is useful in the pharmaceutical field.

This application is based on a patent application No. 2023-069661 filed in Japan (filing date: April 20, 2023), the contents of which are incorporated in full herein.

## Claims

1. A glycolipid derivative (I) represented by the following formula (I): 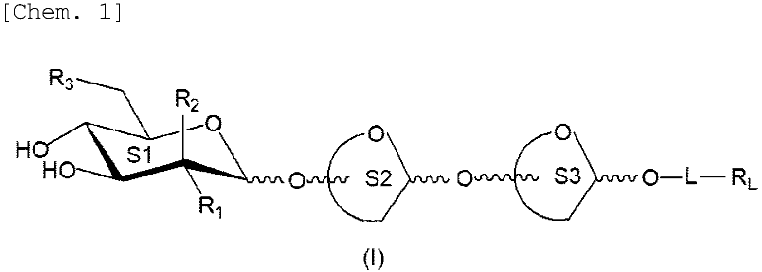
wherein one of R₁ and R₂ is a hydroxy group or a C₁₋₆ alkoxy group, and the other is a hydrogen atom,
R₃ is a hydrogen atom, a hydroxy group, or a C₁₋₆ alkoxy group, 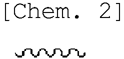
is a single bond (steric configuration is unspecified),
ring S2 and ring S3 are each independently an optionally substituted pyranose or an optionally substituted deoxypyranose, L is a C₆₋₁₄ arylene group, and
R_{L} is an optionally substituted C₃₋₄₀ hydrocarbon group,
or a salt thereof.
(provided that PGL-I, PGL-II, and PGL-III (Rx and Ry of each compound are as shown in Table 1) represented by the following formula (II): 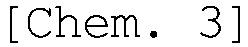 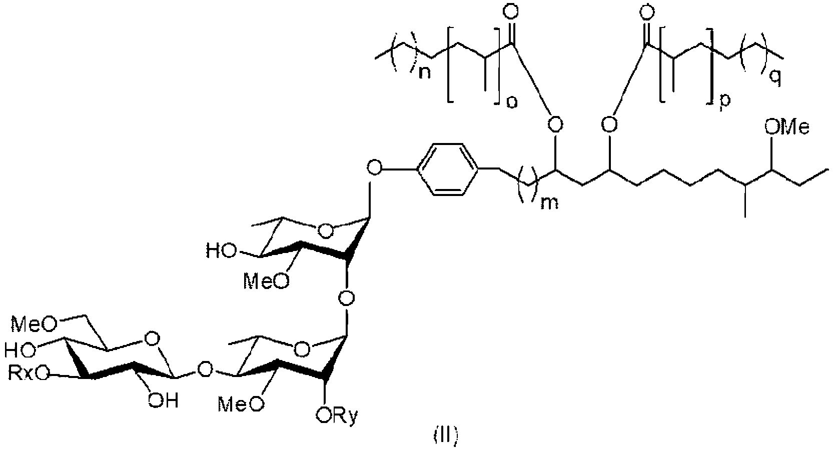
wherein m is an integer of 13 to 21, n is an integer of 15 to 17, o is an integer of 3 to 5, p is an integer of 3 to 5, and q is an integer of 15 to 17
are excluded from the glycolipid derivative (I)
**[Table 1]**
| glycolipid derivative | Rx | Ry |
|---|---|---|
| PGL-I | methyl group | methyl group |
| PGL-II | methyl group | hydrogen atom |
| PGL-III | hydrogen atom | methyl group |

2. The glycolipid derivative (I) according to claim 1,
wherein the terminal sugar represented by ring S1 and the sugar represented by ring S2 are linked by a 1,4 glycosidic bond when viewed from ring S1, or a salt thereof.

3. The glycolipid derivative (I) according to claim 1 or 2, wherein the sugar represented by ring S2 and the sugar represented by ring S3 are linked by a 1,2 or 1,4 glycosidic bond when viewed from ring S2, or a salt thereof.

4. The glycolipid derivative (I) according to any one of claims 1 to 3, which is a derivative represented by the following formula (Ia): 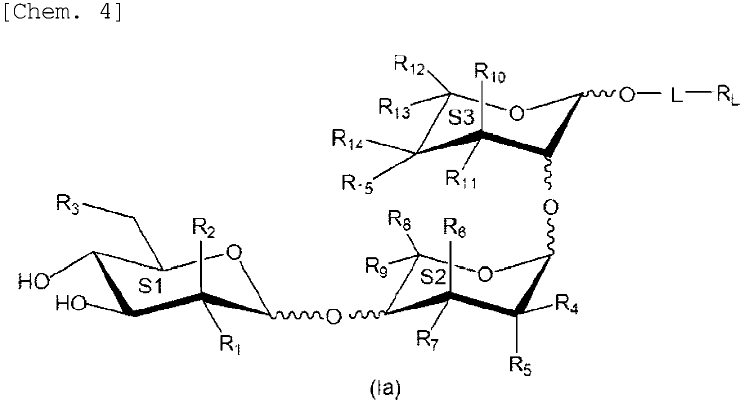 wherein
R₁, R₂, R₃, L, R_{L}, and 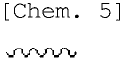
are each the same as the symbols in the glycolipid derivative (I) according to claim 1,
one of R₄ and R₅ is a hydroxy group or a C₁₋₆ alkoxy group, and the other is a hydrogen atom,
one of R₆ and R₇ is a hydroxy group or a C₁₋₆ alkoxy group, and the other is a hydrogen atom,
one of R₈ and R₉ is a group represented by the formula: CH₂-Ra wherein Ra is a hydrogen atom, a hydroxy group, or a C₁₋₆ alkoxy group, and the other is a hydrogen atom,
one of R₁₀ and R₁₁ is a hydroxy group or a C₁₋₆ alkoxy group, and the other is a hydrogen atom,
one of R₁₂ and R₁₃ is a group represented by the formula: CH₂-Ra wherein Ra is a hydrogen atom, a hydroxy group, or a C₁₋₆ alkoxy group, and the other is a hydrogen atom,
one of R₁₄ and R₁₅ is a hydroxy group or a C₁₋₆ alkoxy group, and the other is a hydrogen atom,
or a salt thereof.

5. The glycolipid derivative (I) according to any one of claims 1 to 4, wherein the terminal sugar represented by ring S1 and the sugar represented by ring S2 are linked by a β1,4 glycosidic bond when viewed from ring S1, or a salt thereof.

6. The glycolipid derivative (I) according to any one of claims 1 to 5, wherein one of R₁ and R₂ is a hydroxy group, and the other is a hydrogen atom, or a salt thereof.

7. The glycolipid derivative (I) according to claim 6, wherein R₁ is a hydroxy group and R₂ is a hydrogen atom, or a salt thereof.

8. The glycolipid derivative (I) according to any one of claims 1 to 7, wherein L is a phenylene group, or a salt thereof.

9. The glycolipid derivative (I) according to any one of claims 1 to 8, wherein, in R_{L}, the hydrocarbon group in the optionally substituted C₃₋₄₀ hydrocarbon group is a C₆₋₄₀ hydrocarbon group, or a salt thereof.

10. The glycolipid derivative (I) according to any one of claims 1 to 9, wherein, in R_{L}, the optionally substituted C₃₋₄₀ hydrocarbon group has the same or different 1 to 5 substituents selected from
1) a hydroxy group,
2) an oxo group,
3) an optionally substituted C₃₋₄₀ hydrocarbon group,
4) a C₁₋₆ alkoxy group,
5) an optionally substituted C₃₋₄₀ hydrocarbon-carbonyloxy group,
6) a group containing an epitope sequence,
7) an amino group or an acylamino group,
8) a thiol group, and
9) a halogen atom,
or a salt thereof.

11. A pharmaceutical composition comprising a glycolipid derivative (I') represented by the following formula (I'): 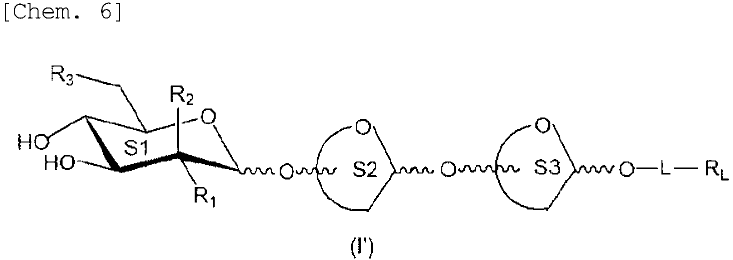
wherein each symbol has the same meaning as the symbol in the glycolipid derivative (I) described in claim 1,
or a salt thereof as an active ingredient (provided that PGL-I and PGL-II (Rx and Ry of each compound are as shown in Table 2) represented by the following formula (II): 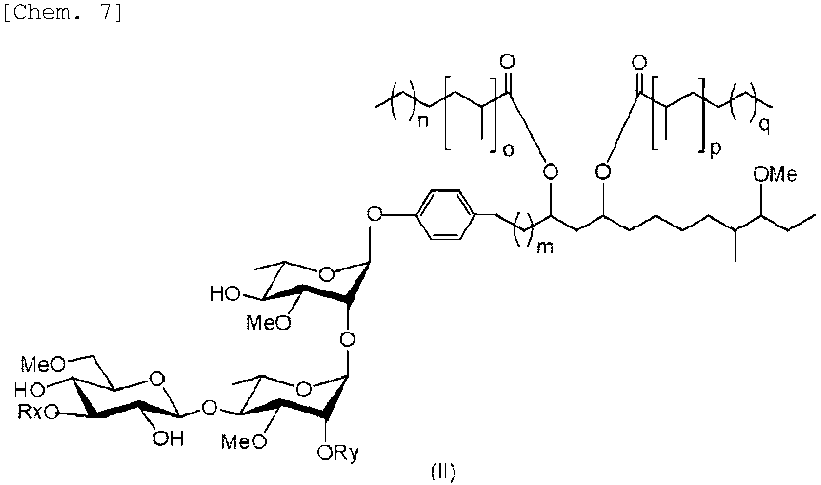
wherein m is an integer of 13 to 21, n is an integer of 15 to 17, o is an integer of 3 to 5, p is an integer of 3 to 5, and q is an integer of 15 to 17
are excluded from the glycolipid derivative (I')
**[Table 2]**
| glycolipid derivative | Rx | Ry |
|---|---|---|
| PGL-I | methyl group | methyl group |
| PGL-II | methyl group | hydrogen atom |

12. The pharmaceutical composition according to claim 11, which is a Mincle activator.

13. The pharmaceutical composition according to claim 11 or 12, which is a TNF production enhancer, an INF-y production enhancer, or an immunostimulant.

14. The pharmaceutical composition according to claim 11 or 12, which is an anticancer agent, or an agent for the prophylaxis and/or treatment of a bacterial infection, a viral infection, or an allergic disease.

15. The pharmaceutical composition according to claim 11 or 12, which is a vaccine adjuvant.
